# EUROPEAN PATENT APPLICATION

(11) **EP 1 473 363 A1**
(43) Date of publication of application: **03.11.2004**
(21) Application number: 03700514.7
(22) Date of filing: 09.01.2003
(51) Int. Cl.: C12N 15/09, C12Q 1/68, G01N 24/08

(54) **METHOD OF MONITORING GENE EXPRESSION**

(30) Priority: 09.01.2002 JP 2002002396
(71) Applicant: Japan Science and Technology Agency, Kawaguchi-shi, Saitama 332-0012 (JP)
(72) Inventor: KOKUBO, Tetsuro, Yokohama-shi, Kanagawa 221-0013 (JP); SHIRAKAWA, Masahiro, Yokohama-shi, Kanagawa 231-0821 (JP); TAME, J.R.H., Yokohama-shi, Kanagawa 230-0051 (JP)
(74) Representative: Harding, Charles Thomas
(86) International application number: PCT/JP2003/000117
(87) International publication number: WO 2003/060117

(57) **Abstract**

The present invention provides a technique by which highly detailed gene expression profiling data for analyzing the gene function can be obtained safely and conveniently. It is a versatile non-destructive and high-resolution visualizing technique by which expression levels of genes can be monitored in real time, and which can be applied in vivo and to deep tissues. For instance, plasmids in which molecules that vary the NMR signal and can be quantified by PHM genes are incorporated downstream from promoters responding to the condition of medium, external stimuli and the like, are introduced into the cells. The level of polyphosphate in deep tissues and the like can then be quantified by ³¹P-NMR, allowing expression profiling of the promoter, both non-destructively and in real time.

## Description

### Technical Field

The present invention relates to a non-destructive method of monitoring gene expression using NMR and a method for screening various types of agents by using the method of monitoring.

### Background Art

Understanding at the molecular level what regulates gene expression in eukaryotes is one of the most important research projects in contemporary biology. Approximately 20 years have passed since R. Roeder succeeded in demonstrating *in vitro* transcription for the first time, numerous experiments using *in vitro* transcription systems have been performed all over the world, and a number of findings relating to the respective elements including the purification/cloning of general transcription factor TFIID by the present inventors have been obtained in the meantime. However, when these findings are applied to biological systems, inexplicable results are still frequently observed. It is presumed that this is not because respective elements are scattered, but because many elements function together as a system *in vivo*. It is thought that *in vitro* experimental systems alone cannot model this behavior, and development of better *in vivo* experimental systems is essential in order to appreciate these regulatory systems.

β-galactosidase and luciferase, which require exogenous substrates, or GFP, which is autofluorescent, have been developed as reporter genes and they have brought useful data at various phases conventionally. The applications for these proteins have been generally limited to transparent organisms, and they have not been useful in the case of opaque samples such as deep tissues of animals. On the other hand, in opaque samples, a method in which transferrin receptor and β-galactosidase are used as reporter genes for high sensitivity imaging by magnetic resonance (MRI) of exogenous genes has been known, but delivery of specific substrate is necessary in both cases. For instance, the former requires fusion of microparticles including iron oxide (called MION) with transferring while the latter requires a gadolinium-ion complex (called EgadMe) containing a galactopyranosyl ring. Further, once these substrates are accumulated *in vivo* they are not degraded easily, therefore they are not suitable for monitoring the expression level of genes in real time. Moreover, it is realistically impossible to make numerous tissue sections, in order to examine gene expression of the whole genome (including as much as 30,000 genes in the case of higher eukaryotes) on a cellular level at very high resolution, and reconstruct it on computer. Apart from a few exceptional organisms having transparent bodies, such as *Brachydanio rerio* (zebrafish) and *Caenorhabditis elegans*, gene expression can only be examined in detail by preexisting methods such as *in situ* hybridization of thin sections. However, as the expression of most of the genes involved in development of the central nervous system is switched on or off extremely rapidly, even analysis with tissue sections is an extremely difficult task. Accordingly, the technique of whole body micro-imaging is becoming increasingly important.

Although the genome sequences of many organisms have now been determined along with the progress of Genome Project, the expression profile of a gene is crucial in understanding the gene function. It is not widely known that promoter region is essential in specifying the temporal and spatial control of gene transcription, and therefore provides extremely valuable data for analyzing the function of proteins encoded downstream. Although it is natural that several genes relating to the same phenomena are expressed in the same cells, there are few cases in which these kinds of data are actually used in discovering the function. This is because the expression of very few genes has been determined at a cellular level of detail. Consequently, it is essential in comprehending the entire biological system to collect the expression data of as many genes as possible, and construct the database of expression profile hence.

As previously mentioned, in opaque samples such as deep tissues of animals, the method of measuring by MRI using transferrin receptors and β-galactosidase genes is known, but both reporters require specific substrates, which are accumulated *in vivo* and degraded slowly, therefore they are not suitable for monitoring expression level of gene in real time, further it is extremely difficult in practice to distribute substrates to all the cells evenly. Besides, non-destructive methods of quantifying that can be applied to deep tissues, µPET (micro-positron emission tomography), SPECT (single-photon emission computed tomography) and the like are known, but their spatial resolution is around one millimetre, and insufficient for visualizing a single cell. The development of a versatile visualizing technique is therefore necessary *in vivo*.

The subject of the present invention is to provide a technique by which the most important gene expression profiling data for analyzing gene function can be obtained safely and conveniently, i. e. a non-destructive and high-resolution visualization *in vivo* technique with high versatility, by which expression levels of genes can be monitored in real time, and which can be applied to deep tissues and the like.

Promoter structure is important in determining the transcription initiation site. When the genes on genomes are transcribed, it is thought that multistep responses that cannot be reproduced *in vitro,* such as structural changes within the nucleus, chromosome, template DNA and the like occur successively. In the meantime, though *Saccharomyces cerevisiae* is a simple unicellular organism having only 6,000 genes, it comprises transcription machinery that is approximately equivalent to that of Man, and it is currently the most attractive model organism to elucidate the detailed pattern of gene expression from the perspective of data expression system. A group of genes (PHM genes) relating to the polyphosphate synthetic pathway was identified in *Saccharomyces cerevisiae* recently for the first time as eukaryotes (Molecular Biology of the Cell, Vol. 11, 4309-4321, 2000).

In *Saccharomyces cerevisiae*, it is known that along with the decrease of concentration of phosphate in the medium, transcription factor PHO4 shifts to a hypophosphorylated form from a hyperphosphorylated form. As a result, the intranuclear level of PHO4 increases to activate a set of target genes relating to phosphate metabolism. Though polyphosphate synthetases PHM1-4 were isolated as candidates for target genes of PHO4, analysis showed that these encode membrane proteins which are very similar to each other, and the level of polyphosphate decreases in each of the deleted strains. Accumulation of polyphosphate cannot be seen at all in phm1Δphm2Δ double mutation line, phm3Δ line and phm4Δ line, which strongly suggests that PHM1-4 genes encode the subunits of polyphosphate synthetase, which is thought to function in vacuoles or on vacuolar membranes. The present inventors considered that the aforementioned polyphosphate synthetase (PHM) gene of *Saccharomyces cerevisiae* might be able to be used as a reporter gene, by quantifying polyphosphate, which is known to be significantly accumulated in all organisms. This quantitation can be achieved by ³¹P-NMR directly, or indirectly by measuring the influence on nearby water of iron ions binding to polyphosphate by ¹H-NMR. Plasmids were constructed carrying PHM genes downstream of promoters responding to various conditions (e.g. environmental stimuli), and introduced into a PHM deleted strain of *Saccharomyces cerevisiae*. The intracellular expression level of polyphosphate was quantified by ³¹P-NMR and ¹H-NMR non-destructively and in real time, showing that the intracellular level of polyphosphate in the yeast cells varies depending on the expression level of the PHM genes. The present invention has thus completed.

### Disclosure of the Invention

The present invention relates to a method of monitoring the expression of a chosen gene, wherein accumulation of a molecule that varies a NMR signal and can be quantified by NMR, without the requirement to add an exogenous substrate ("1"); the method of monitoring expression of a chosen gene according to "1", wherein the molecule that varies the NMR signal and can be quantified by NMR is a polyphosphate ("2"); the method of monitoring expression of a chosen gene according to "2", wherein the polyphosphate is a polyphosphate generated by expression of a polyphosphate synthetase gene placed downstream of the chosen gene ("3"); the method of monitoring expression of a chosen gene according to "2", wherein the polyphosphate is a polyphosphate generated by expression of the polyphosphate synthetase gene placed downstream of the chosen gene in-frame ("4"); the method of monitoring expression of a chosen gene according to "1" , wherein the molecule that varies the NMR signal and can be quantified by NMR is a type of cytochrome ("5"); the method of monitoring expression of a chosen gene according to any one of "1" to "5", wherein the quantification by NMR is a non-destructive quantification by NMR ("6"); the method of monitoring expression of a chosen gene according to any one of "1" to "6", wherein the expression of the chosen gene is monitored in real time ("7"); the method of monitoring expression of a chosen gene according to any one of "1" to "7", wherein the expression level of the chosen gene within a cell, a tissue or an organ is detected ("8"); and the method of monitoring expression of a chosen gene according to any one of "1" to "8", wherein the chosen gene is a target gene of a general transcription factor ("9").

The present invention further relates to a method for screening various types of agents, wherein the method of monitoring expression of a chosen gene according to any one of "1" to "9" is used ("10").

### Brief Description of the Drawings

Fig. 1 shows a picture indicating the result of quantifying phosphate (³¹P) NMR spectra in cell populations of wild-type and phm4 knockout (ΔPHM4) *Saccharomyces cerevisiae* (*S. cerevisiae*).
Fig. 2 shows a picture indicating the result of one-dimensional imaging of cells from wild-type *Saccharomyces cerevisiae* (*S. cerevisiae*) by ³¹P-NMR.
Fig. 3 shows a picture indicating the result of quantifying the amount of polyphosphate in cell population of phm4 knockout line (ΔPHM4 (gall-10 PHM4) in which plasmids carrying PHM4 genes downstream of the GAL1 promoter were transformed, wild-type line and phm4 knockout line (ΔPHM4) of *S*. *cerevisiae*, using the same method as that used in Fig 1.
Fig. 4 shows a picture indicating the result of analyzing colonies of wild-type (upper panel) and phm4 knockout (lower panel) *Saccharomyces cerevisiae*, grown on YPD media containing 1 mM FeCl₃, by ¹H-NMR.

### Best Mode for Carrying Out the Invention

The present invention includes any methods of monitoring in which expression is quantified by NMR and no exogenous substrate is added to the cells. For this purpose, molecules that can be detected by NMR through their effects on the signals can be found from those that absorb radio-frequency fields because of the presences of magnetic moments, which are products of nuclear orbital moments and spin moments, intrinsic to their nucleus. In addition to poly-phosphates, cytochromes and ferritins, both of which contain iron-heme groups, are good examples.

As mentioned above, polyphosphate can accumulate in all organisms in significant amounts and can be quantified directly by ³¹P-NMR. Polyphosphate synthetase genes, for instance PHM genes of *Saccharomyces cerevisiae,* specifically. PHM 1-4 genes placed downstream of the chosen gene and in-frame with it allow transcription to be quantified in real time. The polyphosphate generated by this method has a mean length of up to 50 phosphate groups, within the detectable range by NMR. The 10 mer is thought to show the highest sensitivity for ³¹P-NMR. The polymer sizes can be confirmed by staining the cell contents with toluidine blue after polyacrylamide gel-electrophoresis. Importantly, NMR signals derived from polyphosphate can be independently quantified without interference from signals of nucleic acids. DNA sequences of PHM 1-5 genes and amino acid sequences of PHM 1-5 are shown by Seq. I.D. Nos. 1-10. As for the polyphosphate synthetases used, there is no particular restriction as long as the synthetases can generate polyphosphate intracellularly. PPK (polyphosphate kinase) derived from prokaryotic organisms, functional homologues, or orthlogues, other than PHM of the aforementioned Saccharomyces cerevisiae can be used.

As for using cytochromes molecules, in addition to cytochromes b/c₁/c/a₃, cytochrome c oxidase, cytochrome P450, cytochrome b-559, and b-563 can be used but cytochrome b-5 is preferable since its signal is readily measured by NMR. The DNA sequence of cytochrome b-5 gene from rat, the amino acid sequence of the protein, the DNA sequence of the cytochrome b-5 gene from yeast, and the amino acid sequence of the protein are shown by Seq. I.D. No. 11, Seq. I.D. No. 12, Seq. I.D. No. 13, and Seq. I. D. No. 14, respectively for reference. Apart from heme proteins having iron atoms such as, ferritin, hemoglobin, catalase, peroxidase and the like can also be used as a reporter gene, placed downstream in-frame with the chosen genes.

As for which genes may be studied by the method of monitoring described by the present invention, there are no particular restrictions. Target genes of general transcription factors such as TFIID subunit (TAF), and the like, i. e. genes which are targeted by general transcription factors and regulated their expression by general transcription factors for instance RPS5 gene, HIS4 gene, TUB2 gene and the like are suitable examples for which expression profiling data may be determined by the invention described.

The present invention is particularly suitable for real-time non-destructively monitoring of expression of the chosen gene in opaque samples like deep tissues of animals, so as to detect expression level of the chosen genes in cells, tissues or organs. In order to perform monitoring of expression of the chosen gene according the present invention, the PPK (polyphosphate kinase) gene from *Escherichia coli* may be placed downstream of the chosen promoters and the amount of polyphosphate accumulated can be quantified by NMR. Thus PPK may be used a reporter instead of the aforementioned *Saccharomyces cerevisiae* PHM genes. *Escherichia coli* PPK can synthesize polyphosphate from ATP in a single step, unlike the synthetic system of eukaryotic cells. Other polyphosphate synthetic systems from animals can be used as reporter genes.

As for the method for screening various types of agents that regulate the expression of the chosen gene, it is not particularly restricted as long as it is a method for screening using the aforementioned method of monitoring gene expression by NMR. For instance, by bringing cells or tissues either expressing or accumulating molecules that vary NMR signals and which are detectable by NMR into contact with a test substance *in vitro*, quantifying the expression level of the chosen genes by NMR, and comparing/evaluating the expression level with control agents, without test agents, it is possible to screen for substances inhibiting or promoting the expression of the proteins which are translation products of the chosen genes. As for such candidate agents, therapeutic agents against AIDS, leukemia, viral carcinogenesis such as cancer and the like can be exemplified.

As for the NMR apparatus used in the method of monitoring an expression of the chosen gene and the method for screening the various types of agents of the present invention, it is not particularly restricted as long as it varies NMR and can quantify the NMR signal to be measured. Preferably the probe sections will enable high resolution and the bore will be large. NMR apparatus with good magnetic field gradient amplifiers will allow the method of the invention to be applied not only to microorganisms but also to small animals and plants.

The present invention will be explained more specifically with examples below, but the technical scope of the invention is not limited to these examples.

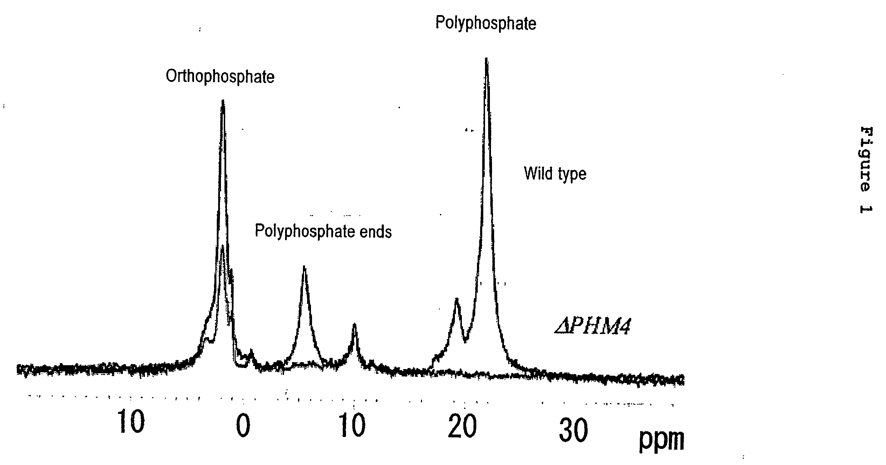

Wild- type line and phm4 knockout line of *Saccharomyces cerevisiae* (*S. cerevisiae*) were grown on YPD medium (Bacto yeast extract [1% w/v], Bacto peptone [2% w/v] and glucose [2% w/v]), and phosphate (³¹P) NMR spectra of the cell population were quantified by using a DRX-500 MHz spectrometer (Bruker). Using literature values, separate signals corresponding to intracellular orthophosphate, and polyphosphate endo and exo groups were identified. The result is shown in Fig. 1. As is apparent from Fig. 1, the most of phosphate was accumulated as polyphoshpate in wild type yeast cells. However, polyphosphate was not present in the phm4 knockout line.

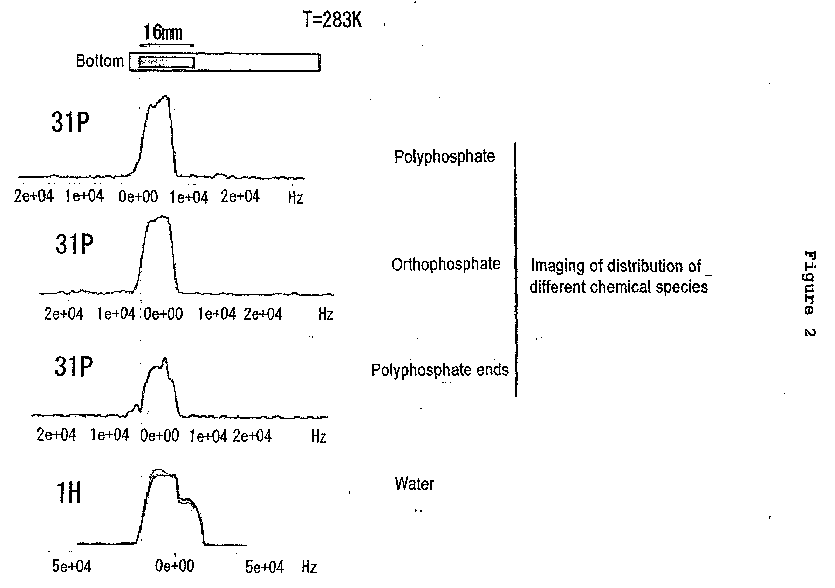

After NMR measuring tubes were filled with cells, one-dimensional imaging (one-dimensional profile) by ³¹P-NMR was taken. The result is shown in Fig. 2. As is apparent from Fig. 2, the profiles could be obtained by using either signal from polyphosphate endo or exo groups, or from orthophosphate. This indicates that imaging of intracellular polyphosphate is possible. On the other hand, it was revealed that one-dimensional profiles using signals derived from water by the frequently used ¹H (hydrogen nuclei) -MRI technique, cannot distinguish the section filled with cells from the section of suspension.

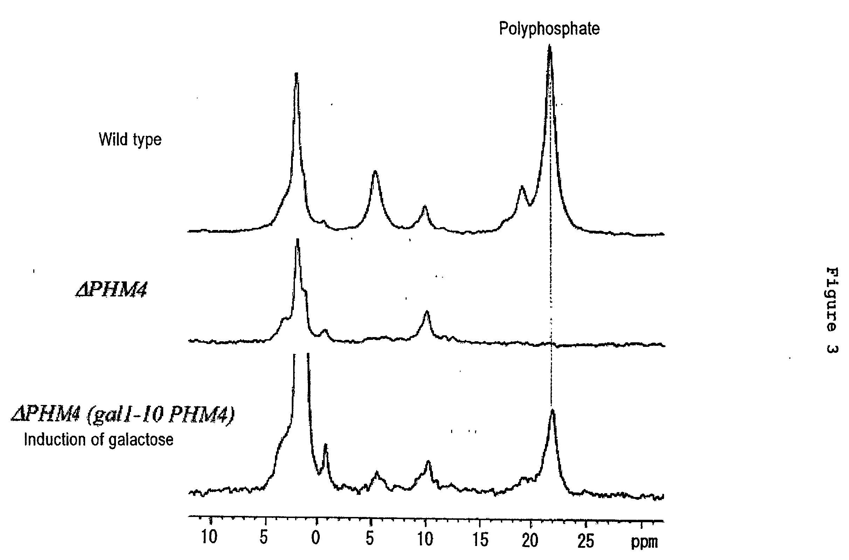

Plasmids carrying PHM4 genes downstream from the GAL1 promoter were used to transform the phm4 knockout line. After transformants were selected on a minimal synthetic medium, they were grown in YPG medium (Bacto yeast extract [1% w/v], Bacto peptone [2% w/v] and galactose [2% w/v]), and the expression of PHM4 genes was induced. The amount of polyphosphate after induction was quantified by the same method as that used in Fig. 1. The result is shown in Fig. 3. As is apparent from Fig. 3, a clear signal due to polyphosphate could be observed. Therefore, the activity of GAL1 promoters could be monitored by ³¹P-NMR signal of polyphosphate.

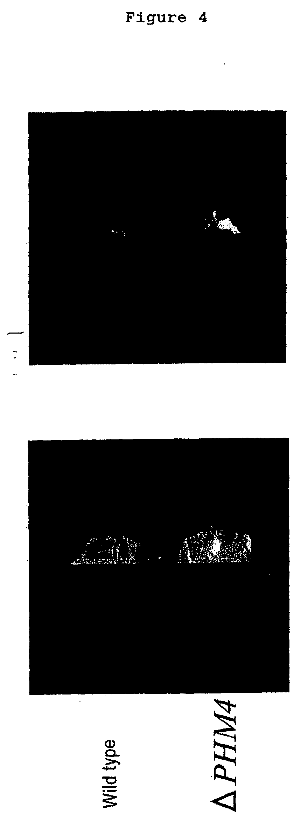

Wild type line (Fig. 4, left) and phm4 knockout line (Fig. 4, right) of *Saccharomyces cerevisiae* (*S. cerevisiae*) were cultured on YPD media with 1 mM of FeCl₃, and colonies were swabbed onto an acrylic plate 3 mm in width and 1 mm in thickness, placed in NMR tubes and imaging of colonies from cross section by ¹H-NMR was performed. The result is shown in Fig. 4. These results revealed that equivalent signals could be detected in both yeast strains by a usual NMR imaging method (Fig. 4, lower panel). The influence of iron ions bound to polyphosphate was emphasized by a modified NMR imaging method; the NMR signals were significantly attenuated in wild yeast by the iron ions (Fig. 4, upper panel), as a result of imaging the aforementioned colonies using the specific technique. It is thought to be possible to delete the signals of wild-type yeast strain completely by improving the conditions further. The expression of PHM4 can be monitored very sensitively with this technique of quantification.

### Industrial Applicability

The present invention is a technique by which highly detailed gene expression profiles for analyzing gene function can be obtained safely and conveniently, i.e. a non-destructive and high-resolution versatile visualizing technique, applicable *in vivo*, and by which the expression level of genes can be monitored in real time, and which can be applied not only to microorganisms and cells but also to deep tissues of mice and the like.

## Claims

1. A method of monitoring expression of a chosen gene, wherein accumulation of a molecule that varies a NMR signal and can be quantified by NMR, without the requirement to add an exogenous substrate.

2. The method of monitoring an expression of a chosen gene according to claim 1, wherein the molecule that varies the NMR signal and can be quantified by NMR is a polyphosphate.

3. The method of monitoring expression of a chosen gene according to claim 2, wherein the polyphosphate is a polyphosphate generated by expression of a polyphosphate synthetase gene placed downstream of the chosen gene.

4. The method of monitoring expression of a chosen gene according to claim 2, wherein the polyphosphate is a polyphosphate generated by expression of the polyphosphate synthetase gene placed downstream of the chosen gene in-frame.

5. The method of monitoring expression of a chosen gene according to claim 1, wherein the molecule that varies the NMR signal and can be quantified by NMR is a type of cytochrome.

6. The method of monitoring expression of a chosen gene according to any one of claims 1 to 5, wherein the quantification by NMR is a non-destructive quantification by NMR.

7. The method of monitoring expression of a chosen gene according to any one of claims 1 to 6, wherein the expression of the chosen gene is monitored in real time.

8. The method of monitoring expression of a chosen gene according to any one of claims 1 to 7, wherein the expression level of the chosen gene within a cell, a tissue or an organ is detected.

9. The method of monitoring expression of a chosen gene according to any one of claims 1 to 8, wherein the chosen gene is a target gene of a general transcription factor.

10. A method for screening various types of agents, wherein the method of monitoring expression of a chosen gene according to any one of claims 1 to 9 is used.


SEQUENCE LISTING

<110> JAPAN SCIENCE AND TECHNOLOGY CORPORATION

<120> Method of monitoring gene expression

<130> YG2002-35PCT

<140>
<141>

<150> JP P2002-002396
<151> 2002-01-09

<160> 14

<170> PatentIn Ver. 2.1

<210> 1
<211> 2487
<212> DNA
<213> Saccharomyces cerevisiae

<220>
<221> CDS
<222> (1)..(2484)

<400> 1
atg ctg ttt gga gtg aag ttg gct aat gag gtt tat cct cct tgg aag 48
Met Leu Phe Gly Val Lys Leu Ala Asn Glu Val Tyr Pro Pro Trp Lys
1               5                   10                  15

ggt tct tat att aat tat gag ggc ttg aaa aaa ttc ctg aag gaa gat 96
Gly Ser Tyr Ile Asn Tyr Glu Gly Leu Lys Lys Phe Leu Lys Glu Asp
            20                  25                  30

agc gtg aag gat gga agt aac gat aag aaa gca cgc tgg gac gat tca 144
Ser Val Lys Asp Gly Ser Asn Asp Lys Lys Ala Arg Trp Asp Asp Ser
        35                  40                  45

gat gaa tcc aag ttt gtg gaa gag ttg gat aag gaa ctt gaa aaa gtc 192
Asp Glu Ser Lys Phe Val Glu Glu Leu Asp Lys Glu Leu Glu Lys Val
    50                  55                  60

tat ggt ttt caa cta aaa aag tac aat aac ttg atg gag aga ttg tcc 240
Tyr Gly Phe Gln Leu Lys Lys Tyr Asn Asn Leu Met Glu Arg Leu Ser
65                  70                  75                  80

cat ctg gag aaa caa aca gat acg gaa gca gcc ata aag gcc ttg gac 288
His Leu Glu Lys Gln Thr Asp Thr Glu Ala Ala Ile Lys Ala Leu Asp
                85                  90                  95

gct gat gca ttc caa cgt gta ttg gag gaa ctg tta agc gag tct acc 336
Ala Asp Ala Phe Gln Arg Val Leu Glu Glu Leu Leu Ser Glu Ser Thr
            100                 105                 110

gaa tta gac aat ttt aag aga ttg aac ttt act ggg ttt gct aag att 384
Glu Leu Asp Asn Phe Lys Arg Leu Asn Phe Thr Gly Phe Ala Lys Ile
        115                 120                 125

gtt aag aaa cat gac aag cta tat cca aag tat cca tct gtt aaa tct 432
Val Lys Lys His Asp Lys Leu Tyr Pro Lys Tyr Pro Ser Val Lys Ser
    130                 135                 140

ttg ttg gaa gtt aga tta aag gaa ttg cct tcc cat tcg gaa gaa tat 480
Leu Leu Glu Val Arg Leu Lys Glu Leu Pro Ser His Ser Glu Glu Tyr
145                 150                 155                 160

tcc cca ttg ttg tat cgt att tca ttt ttg tac aac att ttg aga agt 528
Ser Pro Leu Leu Tyr Arg Ile Ser Phe Leu Tyr Asn Ile Leu Arg Ser
                165                 170                 175

aat ttt aac act gca tct gaa ccc tta gcc agc gct tct aag ttt tct 576
Asn Phe Asn Thr Ala Ser Glu Pro Leu Ala Ser Ala Ser Lys Phe Ser
            180                 185                 190

agc att gtc agc aat gac ata gac atg aat ttc aga agc ttt aaa ttt 624
Ser Ile Val Ser Asn Asp Ile Asp Met Asn Phe Arg Ser Phe Lys Phe
        195                 200                 205

tgg gtt cat aat gac aac tta atg gag gtc aaa aca aga atc ttg aga 672
Trp Val His Asn Asp Asn Leu Met Glu Val Lys Thr Arg Ile Leu Arg
    210                 215                 220

cat ctt ccc gtg ttg gtc tac gcc aat gtt ccc tcc gaa aac gat gac 720
His Leu Pro Val Leu Val Tyr Ala Asn Val Pro Ser Glu Asn Asp Asp
225                 230                 235                 240

ctg gtc aat aga ttc gaa tca gat ata tca aat aat gat gaa att gtg 768
Leu Val Asn Arg Phe Glu Ser Asp Ile Ser Asn Asn Asp Glu Ile Val
                245                 250                 255

ggt agt tcg agc tcc act agt agc gta gaa cat ggc ttg gga gcg cgc 816
Gly Ser Ser Ser Ser Thr Ser Ser Val Glu His Gly Leu Gly Ala Arg
            260                 265                 270

tcc ttc gat cca tta atc aac acg cta tat ttt gac aat gag cat ttt 864
Ser Phe Asp Pro Leu Ile Asn Thr Leu Tyr Phe Asp Asn Glu His Phe
        275                 280                 285

gaa tta tat aac gac aag tta tta aag tta aat tca gca cct act tta 912
Glu Leu Tyr Asn Asp Lys Leu Leu Lys Leu Asn Ser Ala Pro Thr Leu
    290                 295                 300

aga tta agg tgg act ggc cag tta tct gat aag ccg gat att ttc ttg 960
Arg Leu Arg Trp Thr Gly Gln Leu Ser Asp Lys Pro Asp Ile Phe Leu
305                 310                 315                 320

gaa aag aaa act ctt att gaa gac gaa gcc act ggg aag tct gaa ttt 1008
Glu Lys Lys Thr Leu Ile Glu Asp Glu Ala Thr Gly Lys Ser Glu Phe
                325                 330                 335

gat cta act aaa ttg caa ttg aaa caa aaa ttc att aac ggg ttt att 1056
Asp Leu Thr Lys Leu Gln Leu Lys Gln Lys Phe Ile Asn Gly Phe Ile
            340                 345                 350

ttc gaa ggt gat aag aaa ttt aaa gaa caa act ttg aaa aag tta aaa 1104
Phe Glu Gly Asp Lys Lys Phe Lys Glu Gln Thr Leu Lys Lys Leu Lys
        355                 360                 365

gaa agt ggt acg gca ggg aga gac ctg gaa agg tta gaa gaa gat ttc 1152
Glu Ser Gly Thr Ala Gly Arg Asp Leu Glu Arg Leu Glu Glu Asp Phe
    370                 375                 380

tct gag att caa aac ttt att atc aag aat gaa ttg caa cca gtt ttt 1200
Ser Glu Ile Gln Asn Phe Ile Ile Lys Asn Glu Leu Gln Pro Val Phe
385                 390                 395                 400

aga act gtt tac acc aga act gct ttc cag att ccc ggc gat gac aag 1248
Arg Thr Val Tyr Thr Arg Thr Ala Phe Gln Ile Pro Gly Asp Asp Lys
                405                 410                 415

ata aga gta acc att gac tct aat att gta ttc atc aag gag gat tca 1296
Ile Arg Val Thr Ile Asp Ser Asn Ile Val Phe Ile Lys Glu Asp Ser
            420                 425                 430

ttc gac aga gag cgt cca att aga gac cct aat acc tgg cat aga act 1344
Phe Asp Arg Glu Arg Pro Ile Arg Asp Pro Asn Thr Trp His Arg Thr
        435                 440                 445

gat att gat gcc aat gtt gca aat ccc ttg aaa ttc ctg aga gga ggc 1392
Asp Ile Asp Ala Asn Val Ala Asn Pro Leu Lys Phe Leu Arg Gly Gly
    450                 455                 460

gag tat gct aag ttt cct tat tca gta atg gaa att aaa gtg aaa agt 1440
Glu Tyr Ala Lys Phe Pro Tyr Ser Val Met Glu Ile Lys Val Lys Ser
465                 470                 475                 480

tca tta gat tct tcg atg tct gcc agt tct atg att tct aat gta aaa 1488
Ser Leu Asp Ser Ser Met Ser Ala Ser Ser Met Ile Ser Asn Val Lys
                485                 490                 495

ctg cct aaa aag cat ggt caa tgg ctg aac gat ttg aca aat tct cat 1536
Leu Pro Lys Lys His Gly Gln Trp Leu Asn Asp Leu Thr Asn Ser His
            500                 505                 510

ttg gtc aaa gaa att cca aag ttt tct atc ttt gtg caa ggt gtg gca 1584
Leu Val Lys Glu Ile Pro Lys Phe Ser Ile Phe Val Gln Gly Val Ala
        515                 520                 525

tca ttg tat gga gat gat gaa aaa tta gat atc tta cca ttt tgg tta 1632
Ser Leu Tyr Gly Asp Asp Glu Lys Leu Asp Ile Leu Pro Phe Trp Leu
    530                 535                 540

cca gat ttg gaa aca gat att aga cag gat cct aag caa gca tat gag 1680
Pro Asp Leu Glu Thr Asp Ile Arg Gln Asp Pro Lys Gln Ala Tyr Glu
545                 550                 555                 560

gag gaa aag aaa aaa ctg ttg aaa caa aaa gag ata caa aag aaa att 1728
Glu Glu Lys Lys Lys Leu Leu Lys Gln Lys Glu Ile Gln Lys Lys Ile
                565                 570                 575

gat gga atg aga agg ctt tcc aac tta aaa gag cct caa cat caa gca 1776
Asp Gly Met Arg Arg Leu Ser Asn Leu Lys Glu Pro Gln His Gln Ala
            580                 585                 590

gca gta ccg gta tct caa gag gaa aat gag cgt att acc tct caa ggt 1824
Ala Val Pro Val Ser Gln Glu Glu Asn Glu Arg Ile Thr Ser Gln Gly
        595                 600                 605

gat ttg gag gca gac ggt tca tcc gat gag gaa act gag caa gaa cct 1872
Asp Leu Glu Ala Asp Gly Ser Ser Asp Glu Glu Thr Glu Gln Glu Pro
    610                 615                 620

cat tcg aaa aga tca aag aaa gtt cgg aga aga aaa ccc aag gcc act 1920
His Ser Lys Arg Ser Lys Lys Val Arg Arg Arg Lys Pro Lys Ala Thr
625                 630                 635                 640

ttc ttg aga att ttg gcc ggt aga gat cca aag tta atg ggg gtg gat 1968
Phe Leu Arg Ile Leu Ala Gly Arg Asp Pro Lys Leu Met Gly Val Asp
                645                 650                 655

tct gaa gaa gaa gaa att gaa ttg cca cct ggt gtg aaa aaa cca tta 2016
Ser Glu Glu Glu Glu Ile Glu Leu Pro Pro Gly Val Lys Lys Pro Leu
            660                 665                 670

aat ttg tta aaa aat gct ggt cct gta aac gtg gag gca aag gtt tgg 2064
Asn Leu Leu Lys Asn Ala Gly Pro Val Asn Val Glu Ala Lys Val Trp
        675                 680                 685

ctt gcc aac gaa cgt aca ttt aac aga tgg tta agt gtc acc agt tta 2112
Leu Ala Asn Glu Arg Thr Phe Asn Arg Trp Leu Ser Val Thr Ser Leu
    690                 695                 700

ttg agt gtt ttg acg ttc tca att tat aat tct gtg aag aaa gcc gaa 2160
Leu Ser Val Leu Thr Phe Ser Ile Tyr Asn Ser Val Lys Lys Ala Glu
705                 710                 715                 720

tac ccc act ttg gct aac tac atg gca tac gta tat ttt ggt cta acg 2208
Tyr Pro Thr Leu Ala Asn Tyr Met Ala Tyr Val Tyr Phe Gly Leu Thr
                725                 730                 735

ata ttc tgt gct tta tgg tcc tat tcc att tat atg aaa aga gtt gat 2256
Ile Phe Cys Ala Leu Trp Ser Tyr Ser Ile Tyr Met Lys Arg Val Asp
            740                 745                 750

att att caa caa aga agc ggt caa cat cta gat gca cca ctt ggt cca 2304
Ile Ile Gln Gln Arg Ser Gly Gln His Leu Asp Ala Pro Leu Gly Pro
        755                 760                 765

gtt ttg gtt tct ata gtt tta ttt gtc act tta gtg gtt aat ttt gtt 2352
Val Leu Val Ser Ile Val Leu Phe Val Thr Leu Val Val Asn Phe Val
    770                 775                 780

atg gcg ttt aga aat gca gca aag tct cgt caa gag ttg caa ata cag 2400
Met Ala Phe Arg Asn Ala Ala Lys Ser Arg Gln Glu Leu Gln Ile Gln
785                 790                 795                 800

aat tta gaa gtt cct gaa aga ata cca gaa gta tta agg cca ctt caa 2448
Asn Leu Glu Val Pro Glu Arg Ile Pro Glu Val Leu Arg Pro Leu Gln
                805                 810                 815

aat tat cta ttc aag tta atg ggg cca agc agt gat tag 2487
Asn Tyr Leu Phe Lys Leu Met Gly Pro Ser Ser Asp
            820                 825

<210> 2
<211> 828
<212> PRT

<213> Saccharomyces cerevisiae

<400> 2
Met Leu Phe Gly Val Lys Leu Ala Asn Glu Val Tyr Pro Pro Trp Lys
1               5                   10                  15

Gly Ser Tyr Ile Asn Tyr Glu Gly Leu Lys Lys Phe Leu Lys Glu Asp
            20                  25                  30

Ser Val Lys Asp Gly Ser Asn Asp Lys Lys Ala Arg Trp Asp Asp Ser
        35                  40                  45

Asp Glu Ser Lys Phe Val Glu Glu Leu Asp Lys Glu Leu Glu Lys Val
    50                  55                  60

Tyr Gly Phe Gln Leu Lys Lys Tyr Asn Asn Leu Met Glu Arg Leu Ser
65                  70                  75                  80

His Leu Glu Lys Gln Thr Asp Thr Glu Ala Ala Ile Lys Ala Leu Asp
                85                  90                  95

Ala Asp Ala Phe Gln Arg Val Leu Glu Glu Leu Leu Ser Glu Ser Thr
            100                 105                 110

Glu Leu Asp Asn Phe Lys Arg Leu Asn Phe Thr Gly Phe Ala Lys Ile
        115                 120                 125

Val Lys Lys His Asp Lys Leu Tyr Pro Lys Tyr Pro Ser Val Lys Ser
    130                 135                 140

Leu Leu Glu Val Arg Leu Lys Glu Leu Pro Ser His Ser Glu Glu Tyr
145                 150                 155                 160

Ser Pro Leu Leu Tyr Arg Ile Ser Phe Leu Tyr Asn Ile Leu Arg Ser
                165                 170                 175

Asn Phe Asn Thr Ala Ser Glu Pro Leu Ala Ser Ala Ser Lys Phe Ser
            180                 185                 190

Ser Ile Val Ser Asn Asp Ile Asp Met Asn Phe Arg Ser Phe Lys Phe

        195                 200                 205

Trp Val His Asn Asp Asn Leu Met Glu Val Lys Thr Arg Ile Leu Arg
    210                 215                 220

His Leu Pro Val Leu Val Tyr Ala Asn Val Pro Ser Glu Asn Asp Asp
225                 230                 235                 240

Leu Val Asn Arg Phe Glu Ser Asp Ile Ser Asn Asn Asp Glu Ile Val
                245                 250                 255

Gly Ser Ser Ser Ser Thr Ser Ser Val Glu His Gly Leu Gly Ala Arg
            260                 265                 270

Ser Phe Asp Pro Leu Ile Asn Thr Leu Tyr Phe Asp Asn Glu His Phe
        275                 280                 285

Glu Leu Tyr Asn Asp Lys Leu Leu Lys Leu Asn Ser Ala Pro Thr Leu
    290                 295                 300

Arg Leu Arg Trp Thr Gly Gln Leu Ser Asp Lys Pro Asp Ile Phe Leu
305                 310                 315                 320

Glu Lys Lys Thr Leu Ile Glu Asp Glu Ala Thr Gly Lys Ser Glu Phe
                325                 330                 335

Asp Leu Thr Lys Leu Gln Leu Lys Gln Lys Phe Ile Asn Gly Phe Ile
            340                 345                 350

Phe Glu Gly Asp Lys Lys Phe Lys Glu Gln Thr Leu Lys Lys Leu Lys
        355                 360                 365

Glu Ser Gly Thr Ala Gly Arg Asp Leu Glu Arg Leu Glu Glu Asp Phe
    370                 375                 380

Ser Glu Ile Gln Asn Phe Ile Ile Lys Asn Glu Leu Gln Pro Val Phe
385                 390                 395                 400

Arg Thr Val Tyr Thr Arg Thr Ala Phe Gln Ile Pro Gly Asp Asp Lys
                405                 410                 415

Ile Arg Val Thr Ile Asp Ser Asn Ile Val Phe Ile Lys Glu Asp Ser
            420                 425                 430

Phe Asp Arg Glu Arg Pro Ile Arg Asp Pro Asn Thr Trp His Arg Thr
        435                 440                 445

Asp Ile Asp Ala Asn Val Ala Asn Pro Leu Lys Phe Leu Arg Gly Gly
    450                 455                 460

Glu Tyr Ala Lys Phe Pro Tyr Ser Val Met Glu Ile Lys Val Lys Ser
465                 470                 475                 480

Ser Leu Asp Ser Ser Met Ser Ala Ser Ser Met Ile Ser Asn Val Lys
                485                 490                 495

Leu Pro Lys Lys His Gly Gln Trp Leu Asn Asp Leu Thr Asn Ser His
            500                 505                 510

Leu Val Lys Glu Ile Pro Lys Phe Ser Ile Phe Val Gln Gly Val Ala
        515                 520                 525

Ser Leu Tyr Gly Asp Asp Glu Lys Leu Asp Ile Leu Pro Phe Trp Leu
    530                 535                 540

Pro Asp Leu Glu Thr Asp Ile Arg Gln Asp Pro Lys Gln Ala Tyr Glu
545                 550                 555                 560

Glu Glu Lys Lys Lys Leu Leu Lys Gln Lys Glu Ile Gln Lys Lys Ile
                565                 570                 575

Asp Gly Met Arg Arg Leu Ser Asn Leu Lys Glu Pro Gln His Gln Ala
            580                 585                 590

Ala Val Pro Val Ser Gln Glu Glu Asn Glu Arg Ile Thr Ser Gln Gly
        595                 600                 605

Asp Leu Glu Ala Asp Gly Ser Ser Asp Glu Glu Thr Glu Gln Glu Pro
    610                 615                 620

His Ser Lys Arg Ser Lys Lys Val Arg Arg Arg Lys Pro Lys Ala Thr
625                 630                 635                 640

Phe Leu Arg Ile Leu Ala Gly Arg Asp Pro Lys Leu Met Gly Val Asp
                645                 650                 655

Ser Glu Glu Glu Glu Ile Glu Leu Pro Pro Gly Val Lys Lys Pro Leu
            660                 665                 670

Asn Leu Leu Lys Asn Ala Gly Pro Val Asn Val Glu Ala Lys Val Trp
        675                 680                 685

Leu Ala Asn Glu Arg Thr Phe Asn Arg Trp Leu Ser Val Thr Ser Leu
    690                 695                 700

Leu Ser Val Leu Thr Phe Ser Ile Tyr Asn Ser Val Lys Lys Ala Glu
705                 710                 715                 720

Tyr Pro Thr Leu Ala Asn Tyr Met Ala Tyr Val Tyr Phe Gly Leu Thr
                725                 730                 735

Ile Phe Cys Ala Leu Trp Ser Tyr Ser Ile Tyr Met Lys Arg Val Asp
            740                 745                 750

Ile Ile Gln Gln Arg Ser Gly Gln His Leu Asp Ala Pro Leu Gly Pro
        755                 760                 765

Val Leu Val Ser Ile Val Leu Phe Val Thr Leu Val Val Asn Phe Val
    770                 775                 780

Met Ala Phe Arg Asn Ala Ala Lys Ser Arg Gln Glu Leu Gln Ile Gln
785                 790                 795                 800

Asn Leu Glu Val Pro Glu Arg Ile Pro Glu Val Leu Arg Pro Leu Gln
                805                 810                 815

Asn Tyr Leu Phe Lys Leu Met Gly Pro Ser Ser Asp
            820                 825

<210> 3
<211> 2508
<212> DNA
<213> Saccharomyces cerevisiae

<220>
<221> CDS
<222> (1)..(2505)

<400> 3
atg cta ttt ggt att aaa ctg gct aac gac gta tat cct ccc tgg aag 48
Met Leu Phe Gly Ile Lys Leu Ala Asn Asp Val Tyr Pro Pro Trp Lys
1               5                   10                  15

gat tca tat atc gac tat gaa agg ttg aag aaa tta cta aag gaa agc 96
Asp Ser Tyr Ile Asp Tyr Glu Arg Leu Lys Lys Leu Leu Lys Glu Ser
            20                  25                  30

gtc ata cat gat ggc cgt agt tcc gtg gat agt tgg tcc gaa agg aat 144
Val Ile His Asp Gly Arg Ser Ser Val Asp Ser Trp Ser Glu Arg Asn
        35                  40                  45

gaa tct gat ttt gtg gag gcc ttg gat aaa gaa cta gaa aag gtt tac 192
Glu Ser Asp Phe Val Glu Ala Leu Asp Lys Glu Leu Glu Lys Val Tyr
    50                  55                  60

aca ttt caa att tcc aaa tat aat gct gtc ttg cga aaa tta gat gat 240
Thr Phe Gln Ile Ser Lys Tyr Asn Ala Val Leu Arg Lys Leu Asp Asp
65                  70                  75                  80

tta gag gaa aac act aaa tca gct gaa aaa atc caa aaa ata aat tct 288
Leu Glu Glu Asn Thr Lys Ser Ala Glu Lys Ile Gln Lys Ile Asn Ser
                85                  90                  95

gag cag ttc aaa aac acc tta gaa gaa tgt cta gat gag gct caa aga 336
Glu Gln Phe Lys Asn Thr Leu Glu Glu Cys Leu Asp Glu Ala Gln Arg
            100                 105                 110

ttg gat aac ttt gac agg tta aat ttt act ggg ttt atc aag att gtg 384

Leu Asp Asn Phe Asp Arg Leu Asn Phe Thr Gly Phe Ile Lys Ile Val
        115                 120                 125

aag aaa cac gat aaa tta cat cca aac tat cct tct gta aag tct ctt 432
Lys Lys His Asp Lys Leu His Pro Asn Tyr Pro Ser Val Lys Ser Leu
    130                 135                 140

tta caa gtc aga ttg aaa gaa ctt cct ttc aac aat tca gaa gag tat 480
Leu Gln Val Arg Leu Lys Glu Leu Pro Phe Asn Asn Ser Glu Glu Tyr
145                 150                 155                 160

tct ccc ttg ttg tat agg atc tca tat ctg tac gag ttc ttg aga tca 528
Ser Pro Leu Leu Tyr Arg Ile Ser Tyr Leu Tyr Glu Phe Leu Arg Ser
                165                 170                 175

aat tat gat cat cca aat acg gtg tct aaa tca tta gca agt act tct 576
Asn Tyr Asp His Pro Asn Thr Val Ser Lys Ser Leu Ala Ser Thr Ser
            180                 185                 190

aaa tta tca cat ttt tct aac ctt gaa gac gca agt ttt aaa agc tac 624
Lys Leu Ser His Phe Ser Asn Leu Glu Asp Ala Ser Phe Lys Ser Tyr
        195                 200                 205

aag ttt tgg gtt cat gat gat aat ata atg gag gtt aag gct aga atc 672
Lys Phe Trp Val His Asp Asp Asn Ile Met Glu Val Lys Ala Arg Ile
    210                 215                 220

tta agg cat cta cct gct ttg gtt tat gcg tcg gtt cca aat gaa aat 720
Leu Arg His Leu Pro Ala Leu Val Tyr Ala Ser Val Pro Asn Glu Asn
225                 230                 235                 240

gac gat ttc gtc gat aat cta gaa tca gat gtc cgc gta caa cct gaa 768
Asp Asp Phe Val Asp Asn Leu Glu Ser Asp Val Arg Val Gln Pro Glu
                245                 250                 255

gcg cgc ttg aat att ggt tca aag agt aac agc ctt tct agt gat ggt 816
Ala Arg Leu Asn Ile Gly Ser Lys Ser Asn Ser Leu Ser Ser Asp Gly
            260                 265                 270

aat agc aac caa gat gtt gaa att gga aaa tca aag agc gtt att ttc 864

Asn Ser Asn Gln Asp Val Glu Ile Gly Lys Ser Lys Ser Val Ile Phe
        275                 280                 285

cca cag tca tat gat cca aca atc act aca tta tat ttc gac aac gat 912
Pro Gln Ser Tyr Asp Pro Thr Ile Thr Thr Leu Tyr Phe Asp Asn Asp
    290                 295                 300

ttt ttt gat ttg tac aat aac aga ttg ttg aaa atc agc ggt gca cct 960
Phe Phe Asp Leu Tyr Asn Asn Arg Leu Leu Lys Ile Ser Gly Ala Pro
305                 310                 315                 320

acg ctg aga tta aga tgg atc ggt aaa ctg cta gac aaa cct gac ata 1008
Thr Leu Arg Leu Arg Trp Ile Gly Lys Leu Leu Asp Lys Pro Asp Ile
                325                 330                 335

ttt ttg gaa aag aga act ttt aca gaa aat acc gaa act ggt aat tca 1056
Phe Leu Glu Lys Arg Thr Phe Thr Glu Asn Thr Glu Thr Gly Asn Ser
            340                 345                 350

agt ttt gaa gaa atc aga ttg caa atg aaa gcc aaa ttc atc aac aat 1104
Ser Phe Glu Glu Ile Arg Leu Gln Met Lys Ala Lys Phe Ile Asn Asn
        355                 360                 365

ttt att ttt aaa aat gat ccc agt tac aaa aac tat tta att aat caa 1152
Phe Ile Phe Lys Asn Asp Pro Ser Tyr Lys Asn Tyr Leu Ile Asn Gln
    370                 375                 380

cta aga gaa agg ggt act caa aag gag gaa ctg gaa aaa ctt tct aga 1200
Leu Arg Glu Arg Gly Thr Gln Lys Glu Glu Leu Glu Lys Leu Ser Arg
385                 390                 395                 400

gac ttt gat aac atc caa aac ttt att gtt gag gag aag ttg cag cca 1248
Asp Phe Asp Asn Ile Gln Asn Phe Ile Val Glu Glu Lys Leu Gln Pro
                405                 410                 415

gtt ttg aga gct acc tat aat aga act gct ttc caa att cct gga gat 1296
Val Leu Arg Ala Thr Tyr Asn Arg Thr Ala Phe Gln Ile Pro Gly Asp
            420                 425                 430

caa agt att aga gtc act atc gat tcc aat atc atg tac att aga gag 1344

Gln Ser Ile Arg Val Thr Ile Asp Ser Asn Ile Met Tyr Ile Arg Glu
        435                 440                 445

gat tct ttg gac aaa aat agg ccc att agg aac cct gag aat tgg cac 1392
Asp Ser Leu Asp Lys Asn Arg Pro Ile Arg Asn Pro Glu Asn Trp His
    450                 455                 460

cgt gac gat att gat tcc aat att ccc aac cca ttg agg ttt tta aga 1440
Arg Asp Asp Ile Asp Ser Asn Ile Pro Asn Pro Leu Arg Phe Leu Arg
465                 470                 475                 480

gca gga gaa tat tcg aag ttc ccc tat tct gta atg gag att aag gtt 1488
Ala Gly Glu Tyr Ser Lys Phe Pro Tyr Ser Val Met Glu Ile Lys Val
                485                 490                 495

ata aac caa gat aat tct caa atg cct aat tat gag tgg att aaa gat 1536
Ile Asn Gln Asp Asn Ser Gln Met Pro Asn Tyr Glu Trp Ile Lys Asp
            500                 505                 510

tta act aat tca cat tta gtt aac gaa gtt cca aaa ttt tct ttg tac 1584
Leu Thr Asn Ser His Leu Val Asn Glu Val Pro Lys Phe Ser Leu Tyr
        515                 520                 525

ttg caa ggt gtg gct tca ctg ttt ggt gaa gac gat aaa tat gtc aac 1632
Leu Gln Gly Val Ala Ser Leu Phe Gly Glu Asp Asp Lys Tyr Val Asn
    530                 535                 540

att ttg cca ttc tgg ttg ccc gat tta gaa acc gac atc aga aag aac 1680
Ile Leu Pro Phe Trp Leu Pro Asp Leu Glu Thr Asp Ile Arg Lys Asn
545                 550                 555                 560

cct cag gag gct tac gaa gaa gag aag aaa act tta caa aaa caa aag 1728
Pro Gln Glu Ala Tyr Glu Glu Glu Lys Lys Thr Leu Gln Lys Gln Lys
                565                 570                 575

agc att cat gat aaa ctt gat aat atg aga agg tta tcc aaa atc tct 1776
Ser Ile His Asp Lys Leu Asp Asn Met Arg Arg Leu Ser Lys Ile Ser
            580                 585                 590

gta cca gat gga aag acc act gaa aga caa ggg caa aaa gat cag aat 1824

Val Pro Asp Gly Lys Thr Thr Glu Arg Gln Gly Gln Lys Asp Gln Asn
        595                 600                 605

act cgc cac gtt att gca gat tta gaa gat cac gaa tca tca gat gaa 1872
Thr Arg His Val Ile Ala Asp Leu Glu Asp His Glu Ser Ser Asp Glu
    610                 615                 620

gaa ggt act gca ttg ccc aag aaa tct gca gtc aaa aaa ggg aag aaa 1920
Glu Gly Thr Ala Leu Pro Lys Lys Ser Ala Val Lys Lys Gly Lys Lys
625                 630                 635                 640

ttc aaa acg aat gca gct ttc ttg aaa att ctt gct gga aaa aat att 1968
Phe Lys Thr Asn Ala Ala Phe Leu Lys Ile Leu Ala Gly Lys Asn Ile
                645                 650                 655

tca gaa aat gga aat gat cca tac tcc gac gat aca gac agt gcc tct 2016
Ser Glu Asn Gly Asn Asp Pro Tyr Ser Asp Asp Thr Asp Ser Ala Ser
            660                 665                 670

tct ttc caa tta cct ccg gga gtt aaa aaa cca gtt cac ctt ttg aaa 2064
Ser Phe Gln Leu Pro Pro Gly Val Lys Lys Pro Val His Leu Leu Lys
        675                 680                 685

aat gcc ggt cct gtc aaa gtt gag gca aaa gtt tgg ctc gcc aat gaa 2112
Asn Ala Gly Pro Val Lys Val Glu Ala Lys Val Trp Leu Ala Asn Glu
    690                 695                 700

cgt aca ttc aat aga tgg tta agt gta acc aca ttg ctg agt gta ttg 2160
Arg Thr Phe Asn Arg Trp Leu Ser Val Thr Thr Leu Leu Ser Val Leu
705                 710                 715                 720

acc ttt tct atc tat aat tca gtg caa aaa gcc gaa ttt cca caa cta 2208
Thr Phe Ser Ile Tyr Asn Ser Val Gln Lys Ala Glu Phe Pro Gln Leu
                725                 730                 735

gct gat ctg ttg gcc tat gta tat ttc ttt ttg act ttg ttt tgc gga 2256
Ala Asp Leu Leu Ala Tyr Val Tyr Phe Phe Leu Thr Leu Phe Cys Gly
            740                 745                 750

gta tgg gct tat aga acc tac tta aaa aga tta act ctt att aaa ggt 2304

Val Trp Ala Tyr Arg Thr Tyr Leu Lys Arg Leu Thr Leu Ile Lys Gly
        755                 760                 765

aga agt ggt aag cat ttg gat gca cct gtg gga cct att ttg gtt gca 2352
Arg Ser Gly Lys His Leu Asp Ala Pro Val Gly Pro Ile Leu Val Ala
    770                 775                 780

gtt gta tta atc gtt acc ttg gtt gtt aac ttt agt gtg gct ttt aaa 2400
Val Val Leu Ile Val Thr Leu Val Val Asn Phe Ser Val Ala Phe Lys
785                 790                 795                 800

gag gcc gct agg agg gaa aga gga tta gta aac gtt tcc tcc cag cct 2448
Glu Ala Ala Arg Arg Glu Arg Gly Leu Val Asn Val Ser Ser Gln Pro
                805                 810                 815

tcg tta ccc cgt aca cta aaa cca att caa gat ttt atc ttc aat ttg 2496
Ser Leu Pro Arg Thr Leu Lys Pro Ile Gln Asp Phe Ile Phe Asn Leu
            820                 825                 830

gtt ggg gaa taa 2508
Val Gly Glu
        835

<210> 4
<211> 835
<212> PRT
<213> Saccharomyces cerevisiae

<400> 4
Met Leu Phe Gly Ile Lys Leu Ala Asn Asp Val Tyr Pro Pro Trp Lys
1               5                   10                  15

Asp Ser Tyr Ile Asp Tyr Glu Arg Leu Lys Lys Leu Leu Lys Glu Ser
            20                  25                  30

Val Ile His Asp Gly Arg Ser Ser Val Asp Ser Trp Ser Glu Arg Asn
        35                  40                  45

Glu Ser Asp Phe Val Glu Ala Leu Asp Lys Glu Leu Glu Lys Val Tyr

50                  55                  60

Thr Phe Gln Ile Ser Lys Tyr Asn Ala Val Leu Arg Lys Leu Asp Asp
65                  70                  75                  80

Leu Glu Glu Asn Thr Lys Ser Ala Glu Lys Ile Gln Lys Ile Asn Ser
                85                  90                  95

Glu Gln Phe Lys Asn Thr Leu Glu Glu Cys Leu Asp Glu Ala Gln Arg
            100                 105                 110

Leu Asp Asn Phe Asp Arg Leu Asn Phe Thr Gly Phe Ile Lys Ile Val
        115                 120                 125

Lys Lys His Asp Lys Leu His Pro Asn Tyr Pro Ser Val Lys Ser Leu
    130                 135                 140

Leu Gln Val Arg Leu Lys Glu Leu Pro Phe Asn Asn Ser Glu Glu Tyr
145                 150                 155                 160

Ser Pro Leu Leu Tyr Arg Ile Ser Tyr Leu Tyr Glu Phe Leu Arg Ser
                165                 170                 175

Asn Tyr Asp His Pro Asn Thr Val Ser Lys Ser Leu Ala Ser Thr Ser
            180                 185                 190

Lys Leu Ser His Phe Ser Asn Leu Glu Asp Ala Ser Phe Lys Ser Tyr
        195                 200                 205

Lys Phe Trp Val His Asp Asp Asn Ile Met Glu Val Lys Ala Arg Ile
    210                 215                 220

Leu Arg His Leu Pro Ala Leu Val Tyr Ala Ser Val Pro Asn Glu Asn
225                 230                 235                 240

Asp Asp Phe Val Asp Asn Leu Glu Ser Asp Val Arg Val Gln Pro Glu
                245                 250                 255

Ala Arg Leu Asn Ile Gly Ser Lys Ser Asn Ser Leu Ser Ser Asp Gly
            260                 265                 270

Asn Ser Asn Gln Asp Val Glu Ile Gly Lys Ser Lys Ser Val Ile Phe
        275                 280                 285

Pro Gln Ser Tyr Asp Pro Thr Ile Thr Thr Leu Tyr Phe Asp Asn Asp
    290                 295                 300

Phe Phe Asp Leu Tyr Asn Asn Arg Leu Leu Lys Ile Ser Gly Ala Pro
305                 310                 315                 320

Thr Leu Arg Leu Arg Trp Ile Gly Lys Leu Leu Asp Lys Pro Asp Ile
                325                 330                 335

Phe Leu Glu Lys Arg Thr Phe Thr Glu Asn Thr Glu Thr Gly Asn Ser
            340                 345                 350

Ser Phe Glu Glu Ile Arg Leu Gln Met Lys Ala Lys Phe Ile Asn Asn
        355                 360                 365

Phe Ile Phe Lys Asn Asp Pro Ser Tyr Lys Asn Tyr Leu Ile Asn Gln
    370                 375                 380

Leu Arg Glu Arg Gly Thr Gln Lys Glu Glu Leu Glu Lys Leu Ser Arg
385                 390                 395                 400

Asp Phe Asp Asn Ile Gln Asn Phe Ile Val Glu Glu Lys Leu Gln Pro
                405                 410                 415

Val Leu Arg Ala Thr Tyr Asn Arg Thr Ala Phe Gln Ile Pro Gly Asp
            420                 425                 430

Gln Ser Ile Arg Val Thr Ile Asp Ser Asn Ile Met Tyr Ile Arg Glu
        435                 440                 445

Asp Ser Leu Asp Lys Asn Arg Pro Ile Arg Asn Pro Glu Asn Trp His
    450                 455                 460

Arg Asp Asp Ile Asp Ser Asn Ile Pro Asn Pro Leu Arg Phe Leu Arg
465                 470                 475                 480

Ala Gly Glu Tyr Ser Lys Phe Pro Tyr Ser Val Met Glu Ile Lys Val
                485                 490                 495

Ile Asn Gln Asp Asn Ser Gln Met Pro Asn Tyr Glu Trp Ile Lys Asp
            500                 505                 510

Leu Thr Asn Ser His Leu Val Asn Glu Val Pro Lys Phe Ser Leu Tyr
        515                 520                 525

Leu Gln Gly Val Ala Ser Leu Phe Gly Glu Asp Asp Lys Tyr Val Asn
    530                 535                 540

Ile Leu Pro Phe Trp Leu Pro Asp Leu Glu Thr Asp Ile Arg Lys Asn
545                 550                 555                 560

Pro Gln Glu Ala Tyr Glu Glu Glu Lys Lys Thr Leu Gln Lys Gln Lys
                565                 570                 575

Ser Ile His Asp Lys Leu Asp Asn Met Arg Arg Leu Ser Lys Ile Ser
            580                 585                 590

Val Pro Asp Gly Lys Thr Thr Glu Arg Gln Gly Gln Lys Asp Gln Asn
        595                 600                 605

Thr Arg His Val Ile Ala Asp Leu Glu Asp His Glu Ser Ser Asp Glu
    610                 615                 620

Glu Gly Thr Ala Leu Pro Lys Lys Ser Ala Val Lys Lys Gly Lys Lys
625                 630                 635                 640

Phe Lys Thr Asn Ala Ala Phe Leu Lys Ile Leu Ala Gly Lys Asn Ile
                645                 650                 655

Ser Glu Asn Gly Asn Asp Pro Tyr Ser Asp Asp Thr Asp Ser Ala Ser
            660                 665                 670

Ser Phe Gln Leu Pro Pro Gly Val Lys Lys Pro Val His Leu Leu Lys
        675                 680                 685

Asn Ala Gly Pro Val Lys Val Glu Ala Lys Val Trp Leu Ala Asn Glu

690                 695                 700

Arg Thr Phe Asn Arg Trp Leu Ser Val Thr Thr Leu Leu Ser Val Leu
705                 710                 715                 720

Thr Phe Ser Ile Tyr Asn Ser Val Gln Lys Ala Glu Phe Pro Gln Leu
                725                 730                 735

Ala Asp Leu Leu Ala Tyr Val Tyr Phe Phe Leu Thr Leu Phe Cys Gly
            740                 745                 750

Val Trp Ala Tyr Arg Thr Tyr Leu Lys Arg Leu Thr Leu Ile Lys Gly
        755                 760                 765

Arg Ser Gly Lys His Leu Asp Ala Pro Val Gly Pro Ile Leu Val Ala
    770                 775                 780

Val Val Leu Ile Val Thr Leu Val Val Asn Phe Ser Val Ala Phe Lys
785                 790                 795                 800

Glu Ala Ala Arg Arg Glu Arg Gly Leu Val Asn Val Ser Ser Gln Pro
                805                 810                 815

Ser Leu Pro Arg Thr Leu Lys Pro Ile Gln Asp Phe Ile Phe Asn Leu
            820                 825                 830

Val Gly Glu
        835

<210> 5
<211> 1947
<212> DNA
<213> Saccharomyces cerevisiae

<220>
<221> CDS
<222> (1)..(1944)

<400> 5
atg aag ttt ggt gag cac ttg agc aag tct ctt att aga cag tac agc    48
Met Lys Phe Gly Glu His Leu Ser Lys Ser Leu Ile Arg Gln Tyr Ser
1               5                   10                  15

tac tat tat att agt tat gat gat ctg aag act gag cta gaa gat aac    96
Tyr Tyr Tyr Ile Ser Tyr Asp Asp Leu Lys Thr Glu Leu Glu Asp Asn
            20                  25                  30

tta tct aag aat aac ggt cag tgg acg caa gaa ttg gaa aca gat ttt    144
Leu Ser Lys Asn Asn Gly Gln Trp Thr Gln Glu Leu Glu Thr Asp Phe
        35                  40                  45

tta gaa tct ttg gag atc gag tta gac aag gtt tac aca ttt tgc aaa    192
Leu Glu Ser Leu Glu Ile Glu Leu Asp Lys Val Tyr Thr Phe Cys Lys
    50                  55                  60

gtt aag cat agt gaa gtt ttt aga cgt gta aaa gag gtt caa gaa caa    240
Val Lys His Ser Glu Val Phe Arg Arg Val Lys Glu Val Gln Glu Gln
65                  70                  75                  80

gtg caa cat act gtt cgt ttg tta gac tcc aat aat cct cct act cag    288
Val Gln His Thr Val Arg Leu Leu Asp Ser Asn Asn Pro Pro Thr Gln
                85                  90                  95

cta gat ttt gag att cta gaa gaa gaa cta agt gat atc att gcc gat    336
Leu Asp Phe Glu Ile Leu Glu Glu Glu Leu Ser Asp Ile Ile Ala Asp
            100                 105                 110

gtt cat gat cta gca aag ttt tcg aga cta aac tac act ggt ttc caa    384
Val His Asp Leu Ala Lys Phe Ser Arg Leu Asn Tyr Thr Gly Phe Gln
        115                 120                 125

aag att atc aag aaa cac gat aag aag aca ggt ttt atc ttg aaa cca    432
Lys Ile Ile Lys Lys His Asp Lys Lys Thr Gly Phe Ile Leu Lys Pro
    130                 135                 140

gtt ttc caa gtt aga tta gac tct aaa cca ttt ttc aag gaa aac tat    480
Val Phe Gln Val Arg Leu Asp Ser Lys Pro Phe Phe Lys Glu Asn Tyr
145                 150                 155                 160

gac gaa cta gtc gtt aaa att tcc caa cta tat gat att gcc aga act 528
Asp Glu Leu Val Val Lys Ile Ser Gln Leu Tyr Asp Ile Ala Arg Thr
                165                 170                 175

tca ggt cgc cca atc aag gga gac tca tct gct ggt ggg aag caa caa 576
Ser Gly Arg Pro Ile Lys Gly Asp Ser Ser Ala Gly Gly Lys Gln Gln
            180                 185                 190

aat ttc gtt aga cag aca aca aag tat tgg gtt cac cct gat aac att 624
Asn Phe Val Arg Gln Thr Thr Lys Tyr Trp Val His Pro Asp Asn Ile
        195                 200                 205

aca gaa ttg aag ctg atc atc ttg aag cat tta cca gtg tta gtc ttc 672
Thr Glu Leu Lys Leu Ile Ile Leu Lys His Leu Pro Val Leu Val Phe
    210                 215                 220

aac act aat aag gaa ttc gaa aga gaa gat tcc gct att act tcg atc 720
Asn Thr Asn Lys Glu Phe Glu Arg Glu Asp Ser Ala Ile Thr Ser Ile
225                 230                 235                 240

tat ttt gat aat gaa aat cta gac ctt tat tat ggc aga tta aga aaa 768
Tyr Phe Asp Asn Glu Asn Leu Asp Leu Tyr Tyr Gly Arg Leu Arg Lys
                245                 250                 255

gat gaa ggt gca gaa gcc cac aga ttg aga tgg tat ggg ggt atg tct 816
Asp Glu Gly Ala Glu Ala His Arg Leu Arg Trp Tyr Gly Gly Met Ser
            260                 265                 270

aca gac acg atc ttt gta gaa aga aag acc cat aga gag gat tgg act 864
Thr Asp Thr Ile Phe Val Glu Arg Lys Thr His Arg Glu Asp Trp Thr
        275                 280                 285

ggt gag aaa tct gtc aag gca aga ttt gcg cta aag gaa cgt cat gtt 912
Gly Glu Lys Ser Val Lys Ala Arg Phe Ala Leu Lys Glu Arg His Val
    290                 295                 300

aat gac ttc tta aag ggt aaa tat act gtt gat caa gta ttt gcc aag 960
Asn Asp Phe Leu Lys Gly Lys Tyr Thr Val Asp Gln Val Phe Ala Lys
305                 310                 315                 320

atg cgt aaa gaa ggc aag aag cca atg aac gaa att gaa aac ttg gag 1008
Met Arg Lys Glu Gly Lys Lys Pro Met Asn Glu Ile Glu Asn Leu Glu
                325                 330                 335

gcg tta gca tcc gaa att caa tac gtc atg tta aag aaa aag ttg aga 1056
Ala Leu Ala Ser Glu Ile Gln Tyr Val Met Leu Lys Lys Lys Leu Arg
            340                 345                 350

cct gta gtg aga tca ttc tac aat aga act gct ttt caa tta cct ggt 1104
Pro Val Val Arg Ser Phe Tyr Asn Arg Thr Ala Phe Gln Leu Pro Gly
        355                 360                 365

gat gca aga gtt cgt atc tcc ctt gat aca gag tta act atg gtg aga 1152
Asp Ala Arg Val Arg Ile Ser Leu Asp Thr Glu Leu Thr Met Val Arg
    370                 375                 380

gaa gac aac ttt gat ggt gtg gat aga act cat aag aat tgg agg aga 1200
Glu Asp Asn Phe Asp Gly Val Asp Arg Thr His Lys Asn Trp Arg Arg
385                 390                 395                 400

act gat att ggt gtc gat tgg cca ttc aag cag cta gat gac aag gat 1248
Thr Asp Ile Gly Val Asp Trp Pro Phe Lys Gln Leu Asp Asp Lys Asp
                405                 410                 415

att tgc cgt ttc cca tat gca gtc ctg gaa gtt aaa ttg caa act caa 1296
Ile Cys Arg Phe Pro Tyr Ala Val Leu Glu Val Lys Leu Gln Thr Gln
            420                 425                 430

cta ggt caa gaa cct cca gag tgg gta cgt gaa tta gtc ggg tct cac 1344
Leu Gly Gln Glu Pro Pro Glu Trp Val Arg Glu Leu Val Gly Ser His
        435                 440                 445

tta gtt gag cca gtc cca aaa ttc tcc aag ttt att cat ggt gtg gct 1392
Leu Val Glu Pro Val Pro Lys Phe Ser Lys Phe Ile His Gly Val Ala
    450                 455                 460

acc tta cta aat gat aaa gtg gat tca atc cca ttt tgg tta cct caa 1440
Thr Leu Leu Asn Asp Lys Val Asp Ser Ile Pro Phe Trp Leu Pro Gln
465                 470                 475                 480

atg gat gtt gat atc agg aaa cct cca cta cca aca aat att gaa ata 1488
Met Asp Val Asp Ile Arg Lys Pro Pro Leu Pro Thr Asn Ile Glu Ile
                485                 490                 495

aca aga ccc ggt aga tcc gat aac gaa gat aac gac ttc gat gag gat 1536
Thr Arg Pro Gly Arg Ser Asp Asn Glu Asp Asn Asp Phe Asp Glu Asp
            500                 505                 510

gat gag gat gac gct gct ttg gtt gct gcc atg aca aac gca ccc ggt 1584
Asp Glu Asp Asp Ala Ala Leu Val Ala Ala Met Thr Asn Ala Pro Gly
        515                 520                 525

aat tct ctt gat ata gaa gaa tct gtt ggt tac ggg gct act tcg gcc 1632
Asn Ser Leu Asp Ile Glu Glu Ser Val Gly Tyr Gly Ala Thr Ser Ala
    530                 535                 540

ccc acc tcc aac act aat cat gtt gtt gag agt gct aat gct gct tat 1680
Pro Thr Ser Asn Thr Asn His Val Val Glu Ser Ala Asn Ala Ala Tyr
545                 550                 555                 560

tat caa aga aag atc agg aat gcc gaa aat cct atc tcc aag aaa tac 1728
Tyr Gln Arg Lys Ile Arg Asn Ala Glu Asn Pro Ile Ser Lys Lys Tyr
                565                 570                 575

tat gaa atc gtg gca ttt ttt gac cac tat ttt aac ggt gat caa ata 1776
Tyr Glu Ile Val Ala Phe Phe Asp His Tyr Phe Asn Gly Asp Gln Ile
            580                 585                 590

tct aaa atc cca aaa ggt act act ttt gat act caa att cgt gcc cca 1824
Ser Lys Ile Pro Lys Gly Thr Thr Phe Asp Thr Gln Ile Arg Ala Pro
        595                 600                 605

ccg gaa aga cga tat gtg tgc cag ttc gtg tgg aac caa aag ttt act 1872
Pro Glu Arg Arg Tyr Val Cys Gln Phe Val Trp Asn Gln Lys Phe Thr
    610                 615                 620

ttg cca ctg aaa gaa cct acc tgt ctt ggt tgt cca tct cga tat tgt 1920
Leu Pro Leu Lys Glu Pro Thr Cys Leu Gly Cys Pro Ser Arg Tyr Cys
625                 630                 635                 640

tgg gcg gtg tct cca cta ctt tat taa                                    1947
Trp Ala Val Ser Pro Leu Leu Tyr
                645

<210> 6
<211> 648
<212> PRT
<213> Saccharomyces cerevisiae

<400> 6
Met Lys Phe Gly Glu His Leu Ser Lys Ser Leu Ile Arg Gln Tyr Ser
 1               5                  10                  15

Tyr Tyr Tyr Ile Ser Tyr Asp Asp Leu Lys Thr Glu Leu Glu Asp Asn
            20                  25                  30

Leu Ser Lys Asn Asn Gly Gln Trp Thr Gln Glu Leu Glu Thr Asp Phe
        35                  40                  45

Leu Glu Ser Leu Glu Ile Glu Leu Asp Lys Val Tyr Thr Phe Cys Lys
    50                  55                  60

Val Lys His Ser Glu Val Phe Arg Arg Val Lys Glu Val Gln Glu Gln
65                  70                  75                  80

Val Gln His Thr Val Arg Leu Leu Asp Ser Asn Asn Pro Pro Thr Gln
                85                  90                  95

Leu Asp Phe Glu Ile Leu Glu Glu Glu Leu Ser Asp Ile Ile Ala Asp
            100                 105                 110

Val His Asp Leu Ala Lys Phe Ser Arg Leu Asn Tyr Thr Gly Phe Gln
        115                 120                 125

Lys Ile Ile Lys Lys His Asp Lys Lys Thr Gly Phe Ile Leu Lys Pro
    130                 135                 140

Val Phe Gln Val Arg Leu Asp Ser Lys Pro Phe Phe Lys Glu Asn Tyr

145                 150                 155                 160

Asp Glu Leu Val Val Lys Ile Ser Gln Leu Tyr Asp Ile Ala Arg Thr
                165                 170                 175

Ser Gly Arg Pro Ile Lys Gly Asp Ser Ser Ala Gly Gly Lys Gln Gln
            180                 185                 190

Asn Phe Val Arg Gln Thr Thr Lys Tyr Trp Val His Pro Asp Asn Ile
        195                 200                 205

Thr Glu Leu Lys Leu Ile Ile Leu Lys His Leu Pro Val Leu Val Phe
    210                 215                 220

Asn Thr Asn Lys Glu Phe Glu Arg Glu Asp Ser Ala Ile Thr Ser Ile
225                 230                 235                 240

Tyr Phe Asp Asn Glu Asn Leu Asp Leu Tyr Tyr Gly Arg Leu Arg Lys
                245                 250                 255

Asp Glu Gly Ala Glu Ala His Arg Leu Arg Trp Tyr Gly Gly Met Ser
            260                 265                 270

Thr Asp Thr Ile Phe Val Glu Arg Lys Thr His Arg Glu Asp Trp Thr
        275                 280                 285

Gly Glu Lys Ser Val Lys Ala Arg Phe Ala Leu Lys Glu Arg His Val
    290                 295                 300

Asn Asp Phe Leu Lys Gly Lys Tyr Thr Val Asp Gln Val Phe Ala Lys
305                 310                 315                 320

Met Arg Lys Glu Gly Lys Lys Pro Met Asn Glu Ile Glu Asn Leu Glu
                325                 330                 335

Ala Leu Ala Ser Glu Ile Gln Tyr Val Met Leu Lys Lys Lys Leu Arg
            340                 345                 350

Pro Val Val Arg Ser Phe Tyr Asn Arg Thr Ala Phe Gln Leu Pro Gly
        355                 360                 365

Asp Ala Arg Val Arg Ile Ser Leu Asp Thr Glu Leu Thr Met Val Arg
    370                 375                 380

Glu Asp Asn Phe Asp Gly Val Asp Arg Thr His Lys Asn Trp Arg Arg
385                 390                 395                 400

Thr Asp Ile Gly Val Asp Trp Pro Phe Lys Gln Leu Asp Asp Lys Asp
                405                 410                 415

Ile Cys Arg Phe Pro Tyr Ala Val Leu Glu Val Lys Leu Gln Thr Gln
            420                 425                 430

Leu Gly Gln Glu Pro Pro Glu Trp Val Arg Glu Leu Val Gly Ser His
        435                 440                 445

Leu Val Glu Pro Val Pro Lys Phe Ser Lys Phe Ile His Gly Val Ala
    450                 455                 460

Thr Leu Leu Asn Asp Lys Val Asp Ser Ile Pro Phe Trp Leu Pro Gln
465                 470                 475                 480

Met Asp Val Asp Ile Arg Lys Pro Pro Leu Pro Thr Asn Ile Glu Ile
                485                 490                 495

Thr Arg Pro Gly Arg Ser Asp Asn Glu Asp Asn Asp Phe Asp Glu Asp
            500                 505                 510

Asp Glu Asp Asp Ala Ala Leu Val Ala Ala Met Thr Asn Ala Pro Gly
        515                 520                 525

Asn Ser Leu Asp Ile Glu Glu Ser Val Gly Tyr Gly Ala Thr Ser Ala
    530                 535                 540

Pro Thr Ser Asn Thr Asn His Val Val Glu Ser Ala Asn Ala Ala Tyr
545                 550                 555                 560

Tyr Gln Arg Lys Ile Arg Asn Ala Glu Asn Pro Ile Ser Lys Lys Tyr
                565                 570                 575

Tyr Glu Ile Val Ala Phe Phe Asp His Tyr Phe Asn Gly Asp Gln Ile
            580                 585                 590

Ser Lys Ile Pro Lys Gly Thr Thr Phe Asp Thr Gln Ile Arg Ala Pro
        595                 600                 605

Pro Glu Arg Arg Tyr Val Cys Gln Phe Val Trp Asn Gln Lys Phe Thr
    610                 615                 620

Leu Pro Leu Lys Glu Pro Thr Cys Leu Gly Cys Pro Ser Arg Tyr Cys
625                 630                 635                 640

Trp Ala Val Ser Pro Leu Leu Tyr
                645

<210> 7
<211> 390
<212> DNA
<213> Saccharomyces cerevisiae

<220>
<221> CDS
<222> (1)..(387)

<400> 7
atg tct tca gca cca tta tta caa aga aca cct ggg aaa aag atc gct     48
Met Ser Ser Ala Pro Leu Leu Gln Arg Thr Pro Gly Lys Lys Ile Ala
  1               5                  10                  15

ttg ccc aca cga gtt gag cca aaa gtg ttc ttt gcc aat gag cgt acc     96
Leu Pro Thr Arg Val Glu Pro Lys Val Phe Phe Ala Asn Glu Arg Thr
             20                  25                  30

ttt ttg tcg tgg ttg aac ttt aca gtt atg ctg gga ggc ctt ggt gta    144
Phe Leu Ser Trp Leu Asn Phe Thr Val Met Leu Gly Gly Leu Gly Val
         35                  40                  45

ggt tta ctg aat ttt ggt gac aag ata ggt agg gtc agt gca gga cta    192

Gly Leu Leu Asn Phe Gly Asp Lys Ile Gly Arg Val Ser Ala Gly Leu
    50                  55                  60

ttt act ttt gtt gcc atg ggt aca atg ata tac gcg ctt gta aca tac 240
Phe Thr Phe Val Ala Met Gly Thr Met Ile Tyr Ala Leu Val Thr Tyr
65                  70                  75                  80

cac tgg aga gct gct gcg att aga cgt aga gga tca ggt cct tat gat 288
His Trp Arg Ala Ala Ala Ile Arg Arg Arg Gly Ser Gly Pro Tyr Asp
                85                  90                  95

gac aga ttg ggg ccc act ttg ttg tgc ttt ttc tta ttg gtt gct gtc 336
Asp Arg Leu Gly Pro Thr Leu Leu Cys Phe Phe Leu Leu Val Ala Val
            100                 105                 110

att atc aac ttt ata tta aga ttg aag tac aat gac gct aac act aag 384
Ile Ile Asn Phe Ile Leu Arg Leu Lys Tyr Asn Asp Ala Asn Thr Lys
        115                 120                 125

tta tga 390
Leu

<210> 8
<211> 129
<212> PRT
<213> Saccharomyces cerevisiae

<400> 8
Met Ser Ser Ala Pro Leu Leu Gln Arg Thr Pro Gly Lys Lys Ile Ala
1               5                   10                  15

Leu Pro Thr Arg Val Glu Pro Lys Val Phe Phe Ala Asn Glu Arg Thr
            20                  25                  30

Phe Leu Ser Trp Leu Asn Phe Thr Val Met Leu Gly Gly Leu Gly Val
        35                  40                  45

Gly Leu Leu Asn Phe Gly Asp Lys Ile Gly Arg Val Ser Ala Gly Leu
    50                  55                  60

Phe Thr Phe Val Ala Met Gly Thr Met Ile Tyr Ala Leu Val Thr Tyr
65                  70                  75                  80

His Trp Arg Ala Ala Ala Ile Arg Arg Arg Gly Ser Gly Pro Tyr Asp
                85                  90                  95

Asp Arg Leu Gly Pro Thr Leu Leu Cys Phe Phe Leu Leu Val Ala Val
            100                 105                 110

Ile Ile Asn Phe Ile Leu Arg Leu Lys Tyr Asn Asp Ala Asn Thr Lys
        115                 120                 125

Leu

<210> 9
<211> 2025
<212> DNA
<213> Saccharomyces cerevisiae

<220>
<221> CDS
<222> (1)..(2022)

<400> 9
atg gtt gta gtg ggt aag agt gaa gtg cgc aat gta agc atg agt agg      48
Met Val Val Val Gly Lys Ser Glu Val Arg Asn Val Ser Met Ser Arg
1               5                   10                  15

ccg aag aag aaa tca ttg att gcc atc ctg tcg acc tgt gtt ttg ttt      96
Pro Lys Lys Lys Ser Leu Ile Ala Ile Leu Ser Thr Cys Val Leu Phe
            20                  25                  30

ttc ctc gtg ttt ata att ggt gct aaa ttt caa tat gtt tcc gtt ttc      144
Phe Leu Val Phe Ile Ile Gly Ala Lys Phe Gln Tyr Val Ser Val Phe
        35                  40                  45

tcc aaa ttc tta gat gac agg ggg gac aac gag tca ctt caa ttg ctc 192
Ser Lys Phe Leu Asp Asp Arg Gly Asp Asn Glu Ser Leu Gln Leu Leu
    50                  55                  60

aat gac ata gaa ttt aca agg cta gga ctg aca cct agg gag cca gta 240
Asn Asp Ile Glu Phe Thr Arg Leu Gly Leu Thr Pro Arg Glu Pro Val
65                  70                  75                  80

att atc aaa gac gta aag aca ggg aag gag aga aaa tta cat gga cgg 288
Ile Ile Lys Asp Val Lys Thr Gly Lys Glu Arg Lys Leu His Gly Arg
                85                  90                  95

ttc ctt cat att acc gat att cat cct gac cct tat tat gtg gaa gga 336
Phe Leu His Ile Thr Asp Ile His Pro Asp Pro Tyr Tyr Val Glu Gly
            100                 105                 110

agc tct att gat gca gtt tgt cac aca gga aaa cca agc aaa aaa aag 384
Ser Ser Ile Asp Ala Val Cys His Thr Gly Lys Pro Ser Lys Lys Lys
        115                 120                 125

gat gtg gca ccc aag ttt ggg aag gca atg tct gga tgt gat tct ccc 432
Asp Val Ala Pro Lys Phe Gly Lys Ala Met Ser Gly Cys Asp Ser Pro
    130                 135                 140

gtt att tta atg gaa gaa act ctg aga tgg att aaa gaa aat ctt aga 480
Val Ile Leu Met Glu Glu Thr Leu Arg Trp Ile Lys Glu Asn Leu Arg
145                 150                 155                 160

gat aag att gat ttt gtc att tgg acg ggt gat aat atc aga cat gac 528
Asp Lys Ile Asp Phe Val Ile Trp Thr Gly Asp Asn Ile Arg His Asp
                165                 170                 175

aat gac cgg aaa cat cca aga aca gaa gca caa att ttt gat atg aac 576
Asn Asp Arg Lys His Pro Arg Thr Glu Ala Gln Ile Phe Asp Met Asn
            180                 185                 190

aat atc gtt gct gat aaa atg aca gaa tta ttt agt gct ggc aat gaa 624
Asn Ile Val Ala Asp Lys Met Thr Glu Leu Phe Ser Ala Gly Asn Glu
        195                 200                 205

gaa gat cca aga gat ttt gat gtg tct gtc att cca agt ctt gga aac 672
Glu Asp Pro Arg Asp Phe Asp Val Ser Val Ile Pro Ser Leu Gly Asn
    210                 215                 220

aat gat gtc ttt cca cat aac atg ttt gca cta gga cca act cta caa 720
Asn Asp Val Phe Pro His Asn Met Phe Ala Leu Gly Pro Thr Leu Gln
225                 230                 235                 240

act aga gaa tat tat agg att tgg aaa aat ttt gtt ccg cag cag cag 768
Thr Arg Glu Tyr Tyr Arg Ile Trp Lys Asn Phe Val Pro Gln Gln Gln
                245                 250                 255

caa aga act ttt gat agg agt gct tca ttt ttg act gaa gtt att cca 816
Gln Arg Thr Phe Asp Arg Ser Ala Ser Phe Leu Thr Glu Val Ile Pro
            260                 265                 270

ggg aag ctt gct gtc ctg tca att aac acg ctg tac tta ttc aag gcc 864
Gly Lys Leu Ala Val Leu Ser Ile Asn Thr Leu Tyr Leu Phe Lys Ala
        275                 280                 285

aat ccg tta gtt gac aat tgt aat tca aaa aaa gaa cca ggt tac caa 912
Asn Pro Leu Val Asp Asn Cys Asn Ser Lys Lys Glu Pro Gly Tyr Gln
    290                 295                 300

ctt tta ctt tgg ttc ggt tat gta tta gaa gaa cta agg agt agg gga 960
Leu Leu Leu Trp Phe Gly Tyr Val Leu Glu Glu Leu Arg Ser Arg Gly
305                 310                 315                 320

atg aaa gta tgg tta agt gga cat gta cct cca atc gca aaa aat ttc 1008
Met Lys Val Trp Leu Ser Gly His Val Pro Pro Ile Ala Lys Asn Phe
                325                 330                 335

gat cag tcg tgc tac gat aag ttc acg cta tgg act cac gaa tac agg 1056
Asp Gln Ser Cys Tyr Asp Lys Phe Thr Leu Trp Thr His Glu Tyr Arg
            340                 345                 350

gat ata atc att gga gga tta tac ggt cac atg aat att gac cac ttc 1104
Asp Ile Ile Ile Gly Gly Leu Tyr Gly His Met Asn Ile Asp His Phe
        355                 360                 365

atc cca acg gat ggt aaa aaa gct aga aaa tcg tta ttg aaa gcc atg 1152
Ile Pro Thr Asp Gly Lys Lys Ala Arg Lys Ser Leu Leu Lys Ala Met
    370                 375                 380

gag caa tcc act cgt gtt caa caa gga gaa gac agt aat gaa gag gac 1200
Glu Gln Ser Thr Arg Val Gln Gln Gly Glu Asp Ser Asn Glu Glu Asp
385                 390                 395                 400

gaa gaa act gaa ttg aat aga att ctg gat cac gct atg gcg gca aaa 1248
Glu Glu Thr Glu Leu Asn Arg Ile Leu Asp His Ala Met Ala Ala Lys
                405                 410                 415

gaa gtt ttt tta atg gga gcg aaa cca tct aac aaa gaa gca tat atg 1296
Glu Val Phe Leu Met Gly Ala Lys Pro Ser Asn Lys Glu Ala Tyr Met
            420                 425                 430

aac acc gtc cgc gac aca tac tat cgg aaa gtg tgg aat aag ctg gaa 1344
Asn Thr Val Arg Asp Thr Tyr Tyr Arg Lys Val Trp Asn Lys Leu Glu
        435                 440                 445

aga gtg gat gaa aaa aat gtt gaa aat gag aag aaa aag aaa gaa aag 1392
Arg Val Asp Glu Lys Asn Val Glu Asn Glu Lys Lys Lys Lys Glu Lys
    450                 455                 460

aag gac aaa aaa aag aaa aag cca atc act aga aaa gag ctc att gaa 1440
Lys Asp Lys Lys Lys Lys Lys Pro Ile Thr Arg Lys Glu Leu Ile Glu
465                 470                 475                 480

cgc tat tcc att gtg aac ata ggt ggt tca gtc att cca act ttc aat 1488
Arg Tyr Ser Ile Val Asn Ile Gly Gly Ser Val Ile Pro Thr Phe Asn
                485                 490                 495

cct tcc ttt agg atc tgg gaa tat aac atc acc gac ata gtg aat gac 1536
Pro Ser Phe Arg Ile Trp Glu Tyr Asn Ile Thr Asp Ile Val Asn Asp
            500                 505                 510

tcc aat ttt gca gtt tcg gag tat aag ccc tgg gat gaa ttt ttc gag 1584
Ser Asn Phe Ala Val Ser Glu Tyr Lys Pro Trp Asp Glu Phe Phe Glu
        515                 520                 525

tca cta aat aag att atg gaa gac tct ttg cta gaa gat gaa atg gac 1632
Ser Leu Asn Lys Ile Met Glu Asp Ser Leu Leu Glu Asp Glu Met Asp
    530                 535                 540

agc agt aat atc gag gtt ggg atc aac cgc gag aag atg ggc gaa aag 1680
Ser Ser Asn Ile Glu Val Gly Ile Asn Arg Glu Lys Met Gly Glu Lys
545                 550                 555                 560

aaa aac aag aag aaa aag aaa aat gac aag act atg cca att gag atg 1728
Lys Asn Lys Lys Lys Lys Lys Asn Asp Lys Thr Met Pro Ile Glu Met
                565                 570                 575

cca gac aaa tac gaa ctc ggc cct gca tat gta ccg cag tta ttc act 1776
Pro Asp Lys Tyr Glu Leu Gly Pro Ala Tyr Val Pro Gln Leu Phe Thr
            580                 585                 590

ccg acg cgt ttc gtc caa ttc tac gcc gac ttg gaa aaa atc aat caa 1824
Pro Thr Arg Phe Val Gln Phe Tyr Ala Asp Leu Glu Lys Ile Asn Gln
        595                 600                 605

gaa tta cat aat tca ttt gtt gaa tct aag gat att ttc agg tat gag 1872
Glu Leu His Asn Ser Phe Val Glu Ser Lys Asp Ile Phe Arg Tyr Glu
    610                 615                 620

ata gaa tat acc tca gac gag aaa cca tac tca atg gat tcg tta aca 1920
Ile Glu Tyr Thr Ser Asp Glu Lys Pro Tyr Ser Met Asp Ser Leu Thr
625                 630                 635                 640

gta gga agt tat ttg gat ctt gca ggc aga tta tac gaa aac aaa cct 1968
Val Gly Ser Tyr Leu Asp Leu Ala Gly Arg Leu Tyr Glu Asn Lys Pro
                645                 650                 655

gca tgg gaa aaa tac gtc gaa tgg tca ttt gcg tct tct gga tat aaa 2016
Ala Trp Glu Lys Tyr Val Glu Trp Ser Phe Ala Ser Ser Gly Tyr Lys
            660                 665                 670

gat gat taa 2025
Asp Asp

<210> 10
<211> 674
<212> PRT
<213> Saccharomyces cerevisiae

<400> 10
Met Val Val Val Gly Lys Ser Glu Val Arg Asn Val Ser Met Ser Arg
1               5                   10                  15

Pro Lys Lys Lys Ser Leu Ile Ala Ile Leu Ser Thr Cys Val Leu Phe
            20                  25                  30

Phe Leu Val Phe Ile Ile Gly Ala Lys Phe Gln Tyr Val Ser Val Phe
        35                  40                  45

Ser Lys Phe Leu Asp Asp Arg Gly Asp Asn Glu Ser Leu Gln Leu Leu
    50                  55                  60

Asn Asp Ile Glu Phe Thr Arg Leu Gly Leu Thr Pro Arg Glu Pro Val
65                  70                  75                  80

Ile Ile Lys Asp Val Lys Thr Gly Lys Glu Arg Lys Leu His Gly Arg
                85                  90                  95

Phe Leu His Ile Thr Asp Ile His Pro Asp Pro Tyr Tyr Val Glu Gly
            100                 105                 110

Ser Ser Ile Asp Ala Val Cys His Thr Gly Lys Pro Ser Lys Lys Lys
        115                 120                 125

Asp Val Ala Pro Lys Phe Gly Lys Ala Met Ser Gly Cys Asp Ser Pro
    130                 135                 140

Val Ile Leu Met Glu Glu Thr Leu Arg Trp Ile Lys Glu Asn Leu Arg
145                 150                 155                 160

Asp Lys Ile Asp Phe Val Ile Trp Thr Gly Asp Asn Ile Arg His Asp
                165                 170                 175

Asn Asp Arg Lys His Pro Arg Thr Glu Ala Gln Ile Phe Asp Met Asn

            180                 185                 190

Asn Ile Val Ala Asp Lys Met Thr Glu Leu Phe Ser Ala Gly Asn Glu
        195                 200                 205

Glu Asp Pro Arg Asp Phe Asp Val Ser Val Ile Pro Ser Leu Gly Asn
    210                 215                 220

Asn Asp Val Phe Pro His Asn Met Phe Ala Leu Gly Pro Thr Leu Gln
225                 230                 235                 240

Thr Arg Glu Tyr Tyr Arg Ile Trp Lys Asn Phe Val Pro Gln Gln Gln
                245                 250                 255

Gln Arg Thr Phe Asp Arg Ser Ala Ser Phe Leu Thr Glu Val Ile Pro
            260                 265                 270

Gly Lys Leu Ala Val Leu Ser Ile Asn Thr Leu Tyr Leu Phe Lys Ala
        275                 280                 285

Asn Pro Leu Val Asp Asn Cys Asn Ser Lys Lys Glu Pro Gly Tyr Gln
    290                 295                 300

Leu Leu Leu Trp Phe Gly Tyr Val Leu Glu Glu Leu Arg Ser Arg Gly
305                 310                 315                 320

Met Lys Val Trp Leu Ser Gly His Val Pro Pro Ile Ala Lys Asn Phe
                325                 330                 335

Asp Gln Ser Cys Tyr Asp Lys Phe Thr Leu Trp Thr His Glu Tyr Arg
            340                 345                 350

Asp Ile Ile Ile Gly Gly Leu Tyr Gly His Met Asn Ile Asp His Phe
        355                 360                 365

Ile Pro Thr Asp Gly Lys Lys Ala Arg Lys Ser Leu Leu Lys Ala Met
    370                 375                 380

Glu Gln Ser Thr Arg Val Gln Gln Gly Glu Asp Ser Asn Glu Glu Asp
385                 390                 395                 400

Glu Glu Thr Glu Leu Asn Arg Ile Leu Asp His Ala Met Ala Ala Lys
                405                 410                 415

Glu Val Phe Leu Met Gly Ala Lys Pro Ser Asn Lys Glu Ala Tyr Met
            420                 425                 430

Asn Thr Val Arg Asp Thr Tyr Tyr Arg Lys Val Trp Asn Lys Leu Glu
        435                 440                 445

Arg Val Asp Glu Lys Asn Val Glu Asn Glu Lys Lys Lys Lys Glu Lys
    450                 455                 460

Lys Asp Lys Lys Lys Lys Lys Pro Ile Thr Arg Lys Glu Leu Ile Glu
465                 470                 475                 480

Arg Tyr Ser Ile Val Asn Ile Gly Gly Ser Val Ile Pro Thr Phe Asn
                485                 490                 495

Pro Ser Phe Arg Ile Trp Glu Tyr Asn Ile Thr Asp Ile Val Asn Asp
            500                 505                 510

Ser Asn Phe Ala Val Ser Glu Tyr Lys Pro Trp Asp Glu Phe Phe Glu
        515                 520                 525

Ser Leu Asn Lys Ile Met Glu Asp Ser Leu Leu Glu Asp Glu Met Asp
    530                 535                 540

Ser Ser Asn Ile Glu Val Gly Ile Asn Arg Glu Lys Met Gly Glu Lys
545                 550                 555                 560

Lys Asn Lys Lys Lys Lys Lys Asn Asp Lys Thr Met Pro Ile Glu Met
                565                 570                 575

Pro Asp Lys Tyr Glu Leu Gly Pro Ala Tyr Val Pro Gln Leu Phe Thr
            580                 585                 590

Pro Thr Arg Phe Val Gln Phe Tyr Ala Asp Leu Glu Lys Ile Asn Gln
        595                 600                 605

Glu Leu His Asn Ser Phe Val Glu Ser Lys Asp Ile Phe Arg Tyr Glu
    610                 615                 620

Ile Glu Tyr Thr Ser Asp Glu Lys Pro Tyr Ser Met Asp Ser Leu Thr
625                 630                 635                 640

Val Gly Ser Tyr Leu Asp Leu Ala Gly Arg Leu Tyr Glu Asn Lys Pro
                645                 650                 655

Ala Trp Glu Lys Tyr Val Glu Trp Ser Phe Ala Ser Ser Gly Tyr Lys
            660                 665                 670

Asp Asp

<210> 11
<211> 405
<212> DNA
<213> Rattus rattus

<220>
<221> CDS
<222> (1)..(402)

<400> 11
atg gcc gag cag tca gac aag gat gtg aag tac tac act ctg gag gag 48
Met Ala Glu Gln Ser Asp Lys Asp Val Lys Tyr Tyr Thr Leu Glu Glu
1               5                   10                  15

att cag aag cac aaa gac agc aag agc acc tgg gtg atc cta cat cat 96
Ile Gln Lys His Lys Asp Ser Lys Ser Thr Trp Val Ile Leu His His
            20                  25                  30

aag gtg tac gat ctg acc aag ttt ctc gaa gag cat cct ggt ggg gaa 144
Lys Val Tyr Asp Leu Thr Lys Phe Leu Glu Glu His Pro Gly Gly Glu
        35                  40                  45

gaa gtc cta aga gag caa gct ggg ggt gat gct act gag aac ttt gag 192

Glu Val Leu Arg Glu Gln Ala Gly Gly Asp Ala Thr Glu Asn Phe Glu
50                  55                  60

gac gtc ggg cac tct acg gat gca cga gaa ctg tcc aaa aca tac atc     240
Asp Val Gly His Ser Thr Asp Ala Arg Glu Leu Ser Lys Thr Tyr Ile
65                  70                  75                  80

atc ggg gag ctc cat cca gat gac aga tca aag ata gcc aag cct tcg     288
Ile Gly Glu Leu His Pro Asp Asp Arg Ser Lys Ile Ala Lys Pro Ser
                85                  90                  95

gaa acc ctt atc act act gtc gag tct aat tcc agt tgg tgg acc aac     336
Glu Thr Leu Ile Thr Thr Val Glu Ser Asn Ser Ser Trp Trp Thr Asn
            100                 105                 110

tgg gtg atc cca gcc atc tca gcc ctg gtg gta gct ctg atg tat cgc     384
Trp Val Ile Pro Ala Ile Ser Ala Leu Val Val Ala Leu Met Tyr Arg
        115                 120                 125

ctc tac atg gca gaa gat taa                                         405
Leu Tyr Met Ala Glu Asp
    130

<210> 12
<211> 134
<212> PRT
<213> Rattus rattus

<400> 12
Met Ala Glu Gln Ser Asp Lys Asp Val Lys Tyr Tyr Thr Leu Glu Glu
1               5                   10                  15

Ile Gln Lys His Lys Asp Ser Lys Ser Thr Trp Val Ile Leu His His
            20                  25                  30

Lys Val Tyr Asp Leu Thr Lys Phe Leu Glu Glu His Pro Gly Gly Glu
        35                  40                  45

Glu Val Leu Arg Glu Gln Ala Gly Gly Asp Ala Thr Glu Asn Phe Glu

50                    55                    60

Asp Val Gly His Ser Thr Asp Ala Arg Glu Leu Ser Lys Thr Tyr Ile
65                  70                  75                  80

Ile Gly Glu Leu His Pro Asp Asp Arg Ser Lys Ile Ala Lys Pro Ser
                85                  90                  95

Glu Thr Leu Ile Thr Thr Val Glu Ser Asn Ser Ser Trp Trp Thr Asn
            100                 105                 110

Trp Val Ile Pro Ala Ile Ser Ala Leu Val Val Ala Leu Met Tyr Arg
        115                 120                 125

Leu Tyr Met Ala Glu Asp
    130

<210> 13
<211> 363
<212> DNA
<213> Saccharomyces cerevisiae

<220>
<221> CDS
<222> (1)..(360)

<400> 13
atg cct aaa gtt tac agt tac caa gaa gtt gcc gaa cac aat ggc cca      48
Met Pro Lys Val Tyr Ser Tyr Gln Glu Val Ala Glu His Asn Gly Pro
1               5                   10                  15

gaa aat ttc tgg att atc atc gat gac aaa gtt tac gat gtt tct caa      96
Glu Asn Phe Trp Ile Ile Ile Asp Asp Lys Val Tyr Asp Val Ser Gln
            20                  25                  30

ttc aaa gat gaa cat cca ggt ggt gat gaa att ata atg gat ttg ggt      144
Phe Lys Asp Glu His Pro Gly Gly Asp Glu Ile Ile Met Asp Leu Gly
        35                  40                  45

gga caa gat gct aca gaa agc ttt gtc gat atc ggt cat tct gac gaa     192
Gly Gln Asp Ala Thr Glu Ser Phe Val Asp Ile Gly His Ser Asp Glu
    50                  55                  60

gca ttg aga cta ctg aaa ggt tta tac att ggt gac gtt gac aag acc     240
Ala Leu Arg Leu Leu Lys Gly Leu Tyr Ile Gly Asp Val Asp Lys Thr
65                  70                  75                  80

agt gag cgc gtt tct gtg gaa aag gta tct acc tct gaa aac caa agt     288
Ser Glu Arg Val Ser Val Glu Lys Val Ser Thr Ser Glu Asn Gln Ser
                85                  90                  95

aaa ggt agt ggt aca ttg gtt gtc ata ttg gcc att tta atg cta ggt     336
Lys Gly Ser Gly Thr Leu Val Val Ile Leu Ala Ile Leu Met Leu Gly
            100                 105                 110

gtt gct tat tat ttg ttg aac gaa taa                                 363
Val Ala Tyr Tyr Leu Leu Asn Glu
        115                 120

<210> 14
<211> 120
<212> PRT
<213> Saccharomyces cerevisiae

<400> 14
Met Pro Lys Val Tyr Ser Tyr Gln Glu Val Ala Glu His Asn Gly Pro
 1               5                  10                  15

Glu Asn Phe Trp Ile Ile Ile Asp Asp Lys Val Tyr Asp Val Ser Gln
            20                  25                  30

Phe Lys Asp Glu His Pro Gly Gly Asp Glu Ile Ile Met Asp Leu Gly
        35                  40                  45

Gly Gln Asp Ala Thr Glu Ser Phe Val Asp Ile Gly His Ser Asp Glu
    50                  55                  60

Ala Leu Arg Leu Leu Lys Gly Leu Tyr Ile Gly Asp Val Asp Lys Thr
65                  70                  75                  80

Ser Glu Arg Val Ser Val Glu Lys Val Ser Thr Ser Glu Asn Gln Ser
                85                  90                  95

Lys Gly Ser Gly Thr Leu Val Val Ile Leu Ala Ile Leu Met Leu Gly
            100                 105                 110

Val Ala Tyr Tyr Leu Leu Asn Glu
        115                 120